(19) Europäisches Patentamt
European Patent Office
Office européen des brevets

(11) **EP 2 943 906 B1**

(12) **EUROPEAN PATENT SPECIFICATION**

(45) Date of publication and mention
of the grant of the patent:
**21.06.2017 Bulletin 2017/25**

(21) Application number: **14735959.0**

(22) Date of filing: **04.07.2014**

(51) Int Cl.:
**G06F 19/22** *(2011.01)* **G06F 19/24** *(2011.01)*

(86) International application number:
**PCT/EP2014/064310**

(87) International publication number:
**WO 2015/004016 (15.01.2015 Gazette 2015/02)**

(54) **TRANSCRIPT DETERMINATION METHOD**

TRANSKRIPTBESTIMMUNGSVERFAHREN

PROCÉDÉ DE DÉTERMINATION DE TRANSCRIPTION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **09.07.2013 EP 13175774
02.06.2014 EP 14170767**

(43) Date of publication of application:
**18.11.2015 Bulletin 2015/47**

(73) Proprietor: **Lexogen GmbH
1030 Vienna (AT)**

(72) Inventor: **TÜRK, Andreas
A-1020 Vienna (AT)**

(74) Representative: **Sonn & Partner Patentanwälte
Riemergasse 14
1010 Wien (AT)**

(56) References cited:
• **BO LI ET AL: "RSEM: accurate transcript
quantification from RNA-Seq data with or without
a reference genome",** BMC BIOINFORMATICS,
BIOMED CENTRAL, LONDON, GB, vol. 12, no. 1,
4 August 2011 (2011-08-04) , page 323,
XP021104619, ISSN: 1471-2105, DOI:
10.1186/1471-2105-12-323 cited in the application
• **ADAM ROBERTS ET AL: "Improving RNA-Seq
expression estimates by correcting for fragment
bias",** GENOME BIOLOGY, BIOMED CENTRAL
LTD., LONDON, GB, vol. 12, no. 3, 16 March 2011
(2011-03-16) , page R22, XP021097620, ISSN:
1465-6906, DOI: 10.1186/GB-2011-12-3-R22 cited
in the application
• **COLE TRAPNELL ET AL: "Transcript assembly
and quantification by RNA-Seq reveals
unannotated transcripts and isoform switching
during cell differentiation",** NATURE
BIOTECHNOLOGY, vol. 28, no. 5, 1 May 2010
(2010-05-01), pages 511-515, XP055091870, ISSN:
1087-0156, DOI: 10.1038/nbt.1621 cited in the
application
• **LI JUN ET AL: "Modeling non-uniformity in
short-read rates in RNA-Seq data",** GENOME
BIOLOGY, BIOMED CENTRAL LTD., LONDON,
GB, vol. 11, no. 5, 11 May 2010 (2010-05-11), page
R50, XP021085605, ISSN: 1465-6906, DOI:
10.1186/GB-2010-11-5-R50 cited in the
application
• **HSIEH WEN-PING ET AL: "Mixture modeling of
transcript abundance classes in natural
populations",** GENOME BIOLOGY, BIOMED
CENTRAL LTD., LONDON, GB, vol. 8, no. 6, 4 June
2007 (2007-06-04), page R98, XP021031261, ISSN:
1465-6906, DOI: 10.1186/GB-2007-8-6-R98

**Description**

**Field of invention**

The present invention relates to providing information of transcript (e.g. mRNA) abundances based on next generation sequencing (NGS) reads.

**Background**

Next generation sequencing technology produces a large amount of short reads when sequencing a nucleic acid sample. An essential step in next generation sequencing is the library preparation or library prep for short. This process takes mRNA or cDNA as input and produces a library of short cDNA fragments, each corresponding to a section of an mRNA molecule. These fragments are then sequenced by an NGS sequencer, usually not in their entirety but partially at their start and/or at their end. This results in short sequences of nucleotides which are called reads and are most commonly stored by the NGS sequencer as sequences of a group of four ASCII characters such as A, C, G, T or 0, 1, 2, 3, representing the nucleobases of the genetic code. In order to infer which mRNA molecules were present in the original sample, the reads are mapped onto a reference genome.

Next generation sequencing has been employed in a variety of genome mapping procedures (US 2013/110410 A1) or DNA identification methods, e.g. by using a mapped genome to associate sequence reads to a certain organism variant (WO 2009/085412 A1).

WO 2009/091798 A1 describes a method for obtaining a profile of a transcriptome of an organism, the method comprising: sequencing one or more cDNA molecules to obtain sequencing reads; aligning each of the sequencing reads to a reference sequence. However, unknown to prior methods a major problem underlying transcriptome analysis using short sequence reads is the alignment step in case of transcript variants, such as isoforms which differ in sequence deviations, such as e.g. strain difference of a gene, point mutations, or splice variants of one protein. It is usually difficult to align short sequence reads correctly to one transcript variant.

The most common method to assemble transcript sequencing data based on sequence reads is the "Cufflinks" method (Trapnell et al. 2010). Cufflinks constructs a parsimonious set of transcripts that "explain" the reads observed in an RNA-Seq experiment. It does so by reducing the comparative assembly problem to a problem in maximum matching in bipartite graphs. In essence, Cufflinks implements a constructive proof of Dilworth's Theorem by constructing a covering relation on the read alignments, and finding a minimum path cover on the directed acyclic graph for the relation. Using this statistical method, Cufflinks can estimate the abundances of the transcript isoforms present in the sample, either using a known reference annotation, or after an ab-initio assembly of the transcripts using only the reference genome. Cufflinks uses a statistical model of paired-end sequencing experiments to derive a likelihood for the abundances of a set of transcripts given a set of fragments. This likelihood function can be shown to have a unique maximum, which Cufflinks finds using a numerical optimization algorithm. The program then multiplies these probabilities to compute the overall likelihood that one would observe the fragments in the experiment, given the proposed abundances on the transcripts. Because Cufflinks' statistical model is linear, the likelihood function has a unique maximum value, which is found by Cufflinks with a numerical optimization algorithm.

Roberts et al.(2011) relates to methods of improving RNA-Seq expression estimates by correcting fragment bias. Wen-Ping et al.(2007) describes mixture modelling of transcript abundance classes in natural populations.

Prior methods failed to differentiate correctly between transcript variants and to obtain the correct transcript amount or abundance in relation to other transcripts. As shown by comparison herein, even the Cufflinks method failed in several experiments to arrive at the correct transcript abundance information.

It is a goal of the present invention to provide improved methods that allow a more accurate assessment of transcript abundances.

**Summary of the invention**

The present invention provides a method of estimating transcript abundances comprising the steps of

　　a) obtaining transcript fragment sequencing data from a potential mixture of transcripts within a genetic locus of interest,
　　b) assigning said fragment sequencing data to genetic coordinates of said locus of interest thereby obtaining a data set of fragment genetic coordinate coverage, said coverage for each genetic coordinate combined forming a coverage envelope curve (also referred to as total coverage histogram or histogram of total),
　　c) setting a number of transcripts of said mixture, wherein pre-annotated sequence data from the genetic locus of interest is obtained and the number of transcripts is set to at least the number of different isoforms, including splice variants counting as different isoforms, expected from said genetic locus of interest,

d) pre-setting a probability distribution function of modelled genetic coverage for each transcript i, with i denoting the numerical identifier for a transcript, wherein said probability distribution function is composed of the mathematical product of a weight factor $\alpha_i$ of said transcript i and the sum of at least 2 probability subfunctions j, with j denoting the numerical identifier for a probability subfunction, each probability subfunction j being independently weighted by a weight factor $\beta_{i,j}$, wherein the probability subfunctions j are aperiodic functions, wherein the probability subfunctions j for a transcript are positioned or shifted in the genetic coordinate to cover the entire length of a transcript with a positive value,

e) adding the probability distribution functions for each transcript to obtain a sum function,

f) fitting the sum function to the coverage envelope curve thereby optimizing the values for $\alpha_i$ and $\beta_{i,j}$ to increase the fit,

g) repeating steps e) and f) until a pre-set convergence criterion has been fulfilled, thereby obtaining the estimated transcript abundance for each transcript of the mixture given by the weight factor $\alpha_i$ as optimized after the convergence criterion has been fulfilled.

The invention further provides a computer program product employing said method, e.g. containing machine code to perform or assist in said methods and steps on a computer. The computer program product can be provided on any kind of memory device. Also provided is a system, e.g. a computer device, programmed to assist in performing the steps of the inventive method. Calculation steps are usually performed without assistance of an operator. Input and setting steps may be assisted by the program or system, e.g. by suggesting an option proposal for the number and type of probability subfunctions in step d). Of course, the program or system may also be performed with default parameters without further input from an operator.

The following detailed description and preferred embodiments apply to all aspects of the invention and can be combined with each other without restriction, except were explicitly indicated. Preferred embodiments and aspects are defined in the claims.

## Detailed disclosure of the invention

The present invention employs a numerical method to obtain transcript abundance information from a sample of transcript fragment sequences.

The method aligns (NGS) reads, generally referred to as transcript fragment sequences, to a reference sequence, such as a reference genome, to obtain genetic coverage information (step b). Prior statistical tools used for this purpose often make unrealistic assumptions about the nature of the observed data and the estimates of the transcript concentrations are therefore inaccurate. Some of the most widely used tools, such as Cufflinks, assume that the distribution of reads along a transcript is uniform, which contradicts current mRNA-Seq protocols. The invention provides a statistical model that is capable of learning simultaneously the bias of the read distribution along a transcript and the transcript abundances. For this purpose the read or fragment distributions of the transcripts are modelled by mixtures of functions which are trained together with the transcript abundances in the fitting step. The methods used in the fitting step can be inferred from prior maximisation or minimisation procedures, such as a maximum likelihood framework with the expectation maximization algorithm. Since the total probability distribution of the reads in the inventive model is a mixture of mixtures, this model is called the $Mix^2$ (read: Mixquare) model. It is shown in the following that the $Mix^2$ model is very versatile and can be adjusted to different structures inherent in the data by optional parameter tying. In particular, the methods used to obtain transcript abundances can be suitable for transcript related probability distributions. In experiments it is shown that the $Mix^2$ model achieves considerably better estimates for the transcript abundances than the statistical model used in the Cufflinks program. Even when starting from inaccurate transcript annotations the $Mix^2$ model can learn the correct annotation from the data and produces abundance estimates far superior to those of the prior art. Due to the superior learning capability during the fitting step, the starting parameters, e.g. selected during the assignment step a) or during the selection of (e.g. random) probability distribution functions in step d), are not crucial. Even the assumed number of transcripts can be different. It may well be that a wrong transcript annotation or transcript number assumption will be corrected by e.g. fitting the probability distribution function for one or more transcripts to an abundance of zero. The weight factor alpha, which can be modelled as a probability, will represent the abundance of a transcript after convergence.

As used herein "genetic locus of interest" does not limit the method to one continuous sequence stretch of a gene on a chromosome. It generally refers to one or more sections of genetic sequences. A genetic locus can be associated with genomic coordinates, providing position information for sequence reads (transcript fragment sequences). A genetic "position" or "coordinate" is used herein to refer to a nucleotide numerically identified on a reference sequence with a certain distance from the start of the reference sequence. A genetic coordinate can be expressed in genomic coordinates or in transcript coordinates if the genetic coordinate is compatible with the transcript, which is the case if the genetic coordinate is located within any of the transcript exons. Compatible genomic coordinates are transformed into transcript coordinates by calculating the relative distance from the transcript start and subtracting the length of the introns preceding the genomic coordinate. Transcript coordinates can be converted into genomic coordinates by reverting this process. Probability distributions defined on the coordinates of one transcript may be converted to probability distributions on the transcript coordinates of another transcript by shrinking, stretching and shifting of the probability subfunctions.

A nucleobase type (e.g. A, T/U, G, C) may or may not be associated with a genomic coordinate or position. Usually, one type of nucleobase is associated with each genetic coordinate for each transcript. However, for each genetic coordinate different overlapping transcripts may differ in the nucleobase constitution since the present invention can be used to differentiate point mutations, i.e. one or more nucleobases may differ between different transcripts, especially if the sample contained nucleic acid molecules of different organisms or of different alleles. Even in the case of possible mismatches between the transcripts, their fragments and a locus of interest, common genetic coordinates can be allocated. Step b), assigning fragment sequences to genetic coordinates, may comprise sequence comparison or alignment between these sequences, which provides a genetic coordinate. A sequence comparison is well known in the art (e.g. by the open source cufflinks method mentioned) comprising the comparison of the nucleotides. The inventive method can be used on data of different strains or organisms with deviating sequences on a given genetic locus of interest, or may be used to differentiate between different splice variants, i.e. different transcripts which can be distinguished by different combinations of exon sequences. Thus mismatches during assignment step a) can be allowed or disallowed. Such mismatches are preferably at most 1, 2, 3, 4, 5, 6, 7, 8, 9, 10 or 11 mismatches per 100 bases.

The present invention can model the abundance of individual transcripts in a mixture of transcripts (e.g. as the relative amount, which corresponds to a probability). The transcript fragment sequencing data may comprise at least 2, preferably 3, 4, 5, 6, 7, 8, 9, 10, or at least or at most 15, at least or at most 20, at least 25, at least or at most 30, at least or at most 40, transcript sequences. The number of transcripts can be selected e.g. in a selection step performed on a nucleic acid sample by e.g. amplifying or removing nucleic acids. Such removed or amplified nucleic acids may contain a common sequence stretch, such as a sequence corresponding to an oligonucleotide probe, anchor sequence or primer sequence of choice. One or more genomic loci may be selected in this way. In particular preferred embodiments of the invention transcript variants of one gene are investigated. Nevertheless in general, "transcript" as used herein may refer to any nucleic acid or its sequence of any gene or gene combination and any variant thereof, in particular mRNA or cDNA sequence variants thereof.

Preferably the transcript fragment sequences of said transcript fragment sequencing data have a length of 5 to 800 nucleotides, preferably of 6 to 600 nucleotides, of 7 to 400 nucleotides, of 8 to 200 nucleotides or of 9 to 150 nucleotides, even more preferred of 10 to 100 nucleotides, especially preferred of 12 to 70 nucleotides.

The number of transcript fragment sequences may be at least or at most 100, at least or at most 500, at least or at most 1000, at least or at most 5000, at least or at most 10000. Preferably at least or at most 10, or at least or at most 20, at least or at most 50, for each transcript. In combination or as an alternative, the number of transcript fragment sequences may be at most 400000, at most 300000, at most 200000, at most 100000 or at most 50000.

The transcript length of at least one or more, e.g. all, transcripts may be e.g. 100 to 1000000 nucleotides, preferably 1000 to 100000 nucleotides or 2000 to 10000 nucleotides.

In preferred embodiments, the genetic locus of interest comprises one or more isoform, e.g. encoding transcript sequences, of one or more genes or genetic elements, preferably comprises at least 2, 3, 4 or more splice variants of one gene or genetic element. It may comprise one or more other splice variants of another gene or genetic element. In addition or alternatively to splice variants, it may comprise different alleles. In preferred embodiments, the gene or genetic element encodes a protein (e.g. mRNA), but also non protein-coding transcripts, such as regulatory or catalytic RNA, including microRNA, snoRNA or rRNA, as well as their precursors, in particular pre-microRNA or pre-rRNA.

As used herein "gene" and "genetic elements" relate to genetic nucleotides with a sequence that is transcribed to form one or more transcripts.

As used herein "isoform" is used to relate to a particular variant of a transcript. Transcripts of one "gene" or "genetic elements" may differ, e.g. in case of splice variants, thus giving rise to different isoforms. Other isoform variations can be caused by different alleles or different sources of the transcript material, e.g. in a mixture of different microorganisms or strains.

The step of setting a number of transcripts comprises obtaining pre-annotated sequence data from the genetic locus of interest and setting the number of transcripts to at least the number of different transcript sequences, including splice variants counting as different transcript sequences, expected from said genetic locus of interest. As said before, the set number of transcripts may exceed the actual number of transcripts (which may or may not be known exactly) since incorrect transcripts may be removed during fitting of the sum function, resulting in one or more weight factors alphas converging to zero. Usually, a genetic locus of interest is known, e.g. from the cDNA generation step of any one of the nucleic acid selection steps. Using annotated genetic data, it is possible to arrive at a starting number for the amount of transcripts, $i_{max}$. For each transcript, an abundance will be modelled by setting the probability distribution function in step d). This function contains a weight factor alpha, which corresponds to the abundance of the transcript in the mixture after convergence of the sum and probability distribution fitting, an iterative process. The mixture of the transcripts is the first mixture of the inventive model. Each alpha can be modified separately during the fitting process. Thus, setting a number of transcripts may be a rough estimate. For example, it may be 2, 3, 4, 5, 6, 7, 8, 9, 10, 11, 12, 13, 14, 15, at least any one of 16, 20, 30, 40 or more.

A second mixture is mathematically contained in each probability distribution function. The probability distribution function is composed of 2 or more (e.g. 2, 3, 4, 5, 6, 7, 8, 9, 10 or more) probability subfunctions or "blocks". It is essential to have at least 2 probability subfunctions in order to be able to model asymmetric read distributions over the length of the transcript (transcript coordinate range). The total amount of probability subfunctions per transcript is $j_{max}$. $J_{max}$ is usually around 4-8. Too high amounts of probability subfunctions may lead to overtraining and reduced computational efficiency.

The probability distribution function is the sum of the probability subfunctions. Noteworthy, the factor alpha weighs the entire probability distribution function and thus each underlying probability subfunction equally. Within the probability distribution function, the probability subfunctions are weighted separately by a weight factor $\beta_{i,j}$ (beta$_{i,j}$). Of note, for each transcript, i.e. each probability distribution function, the betas may be independent or dependent factors ("tied"). In case of independencies, each beta can be individually modified during the fitting step. It is also possible to tie the betas between the different probability distribution functions, such as the first beta, or generally any given beta$_j$, will be the same for each probability distribution function (e.g. beta$1_{1,j}$= beta$2_{,j}$). This reduces the number of modelled weight factors beta to just $j_{max}$, instead of $i_{max}$ x $j_{max}$, which simplifies the fitting process and reduces computational resource usage. This simplification works best if for each transcript a similar fragment coverage distribution can be expected, e.g. in a given sequence portion always larger abundances are expected than in other portions - for two or more, e.g. all, transcripts (the "tied" transcripts). This assumption is usually true for transcripts of about the same or similar length, such as transcripts having a length (in nucleobases) of 0.3 to 3.2, preferably of 0.5 to 2.1, of another tied transcript. Of course, as will be more clear in the following, other parameters of the probability subfunctions can also be tied in a similar fashion between different transcripts or also between the probability subfunctions of a given transcript (e.g. when shifting the entire probability distribution function for a transcript transversally in the genetic coordinate direction). Also, in step d) it is possible to normalize the probability subfunctions of a transcript to about the same maximum height or maximum value (usually modelled as a probability or probability contribution of the part to the total of a probability distribution function) to each other.

According to a further option for tying of parameters, it is also possible to only tie one or more parameter, e.g. one or more beta, in a group of transcripts, i.e. each probability distribution functions - but not through all transcripts, i.e. each probability distribution function. Such a group may be defined by expected similar shapes of the sum functions of the probability distribution functions. E.g. a group may be defined by similar lengths and/or similar GC-content. A similar length is e.g. when all members of the group have a length within +/- 50%, preferably +/- 35%, more preferred +/- 30%, of a mean size of all members. A similar GC-content may e.g. be within +/- 10%, preferably +/- 5%, of a mean GC-content of all members.

"About" as used herein may refer to the same value or a value differing by +/- 10% of the given value.

The sum of the probability subfunctions for a transcript i constitute the probability distribution function for the transcrip I, with the given weight factors. As probability subfunction any mathematical function can be selected, with the requirement that the sum of these probability subfunctions can form a probability function, wherein the probability subfunction is an aperiodic function. Since the probability subfunctions form the basis for a computational model that can be fitted and optimized, simple functions with few parameters are preferred. The probability subfunction may e.g. comprise or consist of 1, 2, 3, 4, 5, 6, 7, 8, 9 or 10 function parameters determining the shape of the function in dependence of the genetic coordinate. The probability subfunctions j are preferably constituted of positive values for each genetic coordinate. Preferably the probability subfunction is a density function or probability function, but is referred to herein as probability subfunction or "block" to distinguish it from the sum of the probability subfunctions, which is referred to as the probability distribution function (for a given transcript). It usually contains a maximum at a certain genetic coordinate and steadily decreases for positive and negative genetic coordinates removed from said maximum. Preferably the probability subfunction has only one maximum. Example probability subfunctions j are selected from a Gaussian function, a square shape function, a triangle shape function, preferably a Gaussian function.

The genetic coordinates or the range thereof is preferably the same for each transcript. To this end the probability distribution function for a transcript can be shrinked or extended in the genetic coordinate direction, or shifted. Of course such a transformation can be reversed to again arrive at the correct source coordinate in the reference sequence, e.g. the genome. However such a transformation may be beneficial for the abundance modelling algorithm.

This shrinking, extending and shifting can be used to obtain or improve the knowledge of the correct (or more likely) transcript length. In a preferment, a transcript length estimate is obtained by this step, which is used to determine the numbers (e.g. amount or concentration) of fragments. Fragment numbers can be estimated from FPKM (fragments per kilo base pair per million reads) values, based on the relationship: FPKM = number of fragments/transcript length (x weight factor). Hence, the number of fragments correlates to: FPKM x transcript length.

Transcript length estimates can be obtained by setting the transcript start and end positions according to the optionally shrunken or extended and/or shifted probability distribution functions of the sum function after convergence of step e). Due to the nature of the sum function being composed of probability distribution functions, which may not have a defined start and end position, but only probability values for that, the start and end can be estimated by e.g. as the genetic coordinate where the partial area up to (for start) or starting from (for end) said genetic coordinate is a fraction of the whole area under the curve (of the sum function or the first or last probability distribution function). This fraction can be a value between 1% to 10%. A skilled practitioner can easily test suitable values to determine such an area cut-off dependent on the shape of the used probably distribution function. It can be easily tested using model nucleic acids with a known start and end position and setting up the inventive function with other start and end positions and letting the inventive algorithm train improved start and end positions as described. Example values used for cut-off values are 0.5%, 1%, 2,%, 3%, 4%, 5%, 6%, 7%, 8%, 9% or 10%.

According to a further option, the fragment length distribution or average fragment length is calculated using the inventive model functions after convergence of step e).

In particular, the genetic coordinate for one or more, preferably each transcript (and its probability distribution function) may correspond to nucleotide positions in a genome, optionally transformed to omit genetic regions not of interest, wherein preferably said genetic regions not of interest do not contain coverage by said transcript fragment sequencing data - such as introns for which no read or sequence fragment aligns. Thus a continuous probability distribution function can be modelled with no intermittent zero distribution. Such regions not of interest can be removed from the individual probability distribution functions and additionally or alternatively also from the coverage envelope curve (to which the sum function of the various probability distribution functions is fitted during each iteration step g)). The envelope curve represents the histogram of the combined sequence data coverage.

Thus preferably the inventive method further comprises a step b2) comprising removing genetic coordinate positions with introns from said coverage envelope curve. This results in obtaining a modified coverage envelope curve, which replaces the use of the coverage envelope curve in step f) and preferably all other steps - except of course where the probability distribution functions are again referred back to the original fragment sequencing data or the genetic or genomic coordinates of the locus of interest.

The genetic coordinate coverage is not required to use the entire sequence information of a read in order to model the abundance. It is also sufficient to use only a limited number of nucleotides of the read sequence, e.g. just the first one or start site information. The fragment genetic coordinate coverage can contain the count of at least one nucleotide for each fragment sequence assigned to a genetic coordinate, preferably wherein at least one nucleotide comprises the fragment start site or the entire fragment sequence. "Comprises" as used herein shall be understood as an open definition, allowing further members as in containing. "Consisting" on the other hand is considered as a closed definition without further elements of the consisting definition feature. Thus "comprising" is a broader definition and contains the "consisting" definition. Any definitions herein using the "comprising" language may also be read with a consisting limitation in a special embodiment of the invention.

The two or more probability subfunctions, which are the components of the probability distribution function, are preferably evenly distributed among the genomic coordinate range of said probability distribution function (of a transcript). In general, preferably the probability subfunctions for a transcript comprise maxima each at a different genetic coordinate. In a particular preferred embodiment of the invention, the maxima of the probability subfunctions for each transcript are separated at least about $1/j_{max}$ times the difference between the first and last nucleotide of the genetic coordinate as assigned to the transcript in step a), optionally after transformation as described above (which may provide a modified genetic coverage, such as after removal of the introns). Thus the probability subfunctions can be distributed evenly along the genetic coordinate covered by a transcript (and hence the probability density function modeling the coverage). Of course, it is also possible to deviate from the optimal even distribution. Thus the maximum location on the genetic coordinate may deviate e.g. 0%-50%, preferably 10%-40%, of the transcript length or the transcript length divided by $j_{max}$, from the even distribution maximum for each probability subfunction.

Thus, in step d) the probability subfunctions for a transcript are positioned or shifted in the genetic coordinate to cover the entire length of a transcript with a positive value.

In preferred embodiments the method comprises determining sequence reads of at least one transcript, preferably mRNA or cDNA, wherein said reads comprise the sequence of fragments of said transcript, to provide said transcript fragment sequencing data. The determination step can be performed by any method known in the art, such as PCR sequencing. Such method include Maxam-Gilbert sequencing, Chain-termination methods, Shotgun sequencing, Bridge PCR, Massively parallel signature sequencing (MPSS), Polony sequencing, pyrosequencing, Illumina (Solexa) sequencing, SOLiD sequencing, Ion semiconductor sequencing, DNA nanoball sequencing, Heliscope single molecule sequencing, Single molecule real time (SMRT) sequencing, Nanopore DNA sequencing, Sequencing by hybridization, Sequencing with mass spectrometry, Microfluidic Sanger sequencing, Microscopy-based techniques, RNAP sequencing, In vitro virus high-throughput sequencing.

The inventive method may alternatively or in addition comprise the step of feeding transcript fragment sequencing data to a computational device capable of performing the inventive method, e.g. in case of using pre-prepared sequence data. Said computation device will then perform the inventive method to arrive at an abundance information with the feeded sequencing data.

The abundance information can be displayed on an output device, such as a video screen, a printer or a computer readable medium, e.g. as described further below.

Fitting in step f) can be performed by the expectation maximization algorithm. The expectation maximization algorithm has been described by Dempster et al., 1977. Of course any other maximization or minimization algorithm can be adapted to fit the inventive sum function to the envelope curve. In essence the weights alpha i and beta i,j - and optionally any further parameters which define the probability subfunctions - are modified to minimize the difference between the sum function (the model) and the coverage envelope curve (the real data based on the fragment sequences), e.g. by varying the parameters of the probability subfunctions. Many algorithms exist to make such a minimization or variations to find a minimum, especially after several iterations by repeating the calculation steps e) and f).

Such an additional parameter of a probability subfunction is e.g. the full width at half maximum value or, especially in case of gauss functions, the standard deviation value (sigma i,j). These are parameters of function width. Preferably the function width at any genetic coordinate separate from the maximum of the probability subfunction (e.g. preferably the full width at half maximum value or standard deviation) for each probability subfunction for a transcript i is about identical, preferably is identical. Alternatively the ratio of the width to the maximum value is about identical, preferably is identical, for each probability subfunction for a transcript i. This "ties" the function width similarly as described above for the betas between the different probability distribution functions of the transcripts.

In general, in preferred embodiments, at least 1, 2, 3, 4, 5, or 6 parameters of the probability subfunctions within the group of probability subfunctions of a transcript and/or within the group of probability subfunctions with the same identifier j for separate transcripts, are tied, e.g. modified in combination with each other, during the fitting step f).

The last step is convergence of the fitting. A convergence criterion can be set by an operator. It can be a maximum adjustment of any one of the parameters $\alpha_i$ or $\beta_{i,j}$ or a combination thereof, e.g. all $\alpha$s or all $\beta$s, or all $\alpha$s and $\beta$s, or a maximum adjustment in the probability distribution function, e.g. as in equation (15) in the examples. For example, a convergence criterion can be if the log likelihood increase according to equation (21) is below 0.5. Any convergence criterion can be selected by an operator to achieve the desired quality.

In a preferment, the inventive method is further combined with other bias reduction methods. E.g. the obtained estimated transcript abundance of step e) is further weighted by a bias factor, thereby obtaining weighted estimated transcript abundance. This bias factor can take into account that certain transcripts are increased or decreased during wet-chemical transcript or fragment generation. Some fragments, for example, fragments starting with C may be overrepresented and are preferable weighted by a bias factor lower than 1 thereby decreasing the weighted estimated transcript abundance in comparison to the estimated transcript abundance of step e). The chemical reason why fragments starting with C are overrepresented lies in primer kinetics: primers anneal better with templates starting with C as compared to templates starting with G or T. Therefore, fragments starting with G and/or T may be weighted by a bias factor greater than the bias factor for fragments starting with C. In general, a skilled practitioner can obtain such bias factors for any parameter where a systematic bias exist, which may depend on any polynucleic acid property, and include an accordant bias factor an weigh the result of step 3) accordingly.

The invention further relates to a computer readable memory device comprising a computer program product for performing a method according to the invention on a computer or for supporting a method according to the invention by a computer, especially any one of steps a), b), b2) c), d), e), f) and g) may be performed on a computer. Any inventive method or step can be performed as computer-implemented method. Even the usually wetchemistry step of determining sequence reads may be assisted by a computer, e.g. to control and obtain data from an automated or semi-automated sequence reader. The computer program product or memory device may also be provided with a read generation component that obtains short reads from a sample, such as a sequencer, preferably a sequencer comprising a computer component. For example, computer readable media can include but are not limited to magnetic storage devices (e.g., hard disk, floppy disk, magnetic strips, ...), optical disks (e.g., compact disk (CD), digital versatile disk (DVD), ...), smart cards, and flash memory devices (e.g., card, stick, key drive, ...).

The present invention is further illustrated by the following figures and examples, without being limited to these embodiments of the invention, with - each element being combinable with any other embodiment of the invention. Especially any one of the formulas provided below can be used separately, individually or in combination to describe or define a step in the inventive method.

**Figures:**

Figure 1: NGS work-flow following the steps of cDNA generation, fragmentation, sequencing, mapping the sequence reads to a reference sequence and analysis of the mapped data set of fragment sequences;

Figure 2: mixture of coverage histograms of two transcripts;

Figure 3, a reference figure: Distribution of fragment start sites assumed by the Cufflinks model;

Figure 4, a reference figure: Coverage assumed by the Cufflinks model;

Figure 5: Coverage of transcript reads on the Uqcrq gene

Figure 6: Genetic coordinate transformation; Decomposition of the total probability $p_{(total)}(r)$ by scaled and shifted versions of the average probability $p_{(avg)}(r)$; total abundance envelope (top), abundance for transcript 1 (middle), transformation of initial genetic coverage for transcript 1 by removing junctions (shift) and scaling to illustrate a modified genetic coverage for transcript 1 (bottom);

Figure 7: Exon structure and junctions of three model transcripts with equal abundance (each being 1/3 of the total) on a genetic coordinate;

Figure 8: Start site probability distributions $p(s(r)|t=i)$ in transcript coordinates for transcripts in Figure 7 after removal of junctions (shift);

Figure 9: Start site probability distributions $p(s(r)|t=i)$ in genome coordinates for transcripts in Figure 7;

Figure 10: Total probability distribution of start sites for transcripts in Figures 7 and 8 with equal abundances $\alpha_i = 1/3$ for i=1,2,3;

Figure 11: Extended start and end sites of transcripts in Figure 7. The model estimates the correct start and end site distributions with the scale and shift parameters lambda i and nu i;

Figure 12: Tying of the sigmas: sigma, an optional further standard deviation (width) parameter in the probability subfunctions, is held constant within each probability subfunction for a transcript i, shown are two transcripts 1 (middle) and 2 (top) with the sum function (below); dashed lines are missing probability subfunctions for modelling a sum function of otherwise evenly distributed probability subfunctions (here Gauss functions) over the genetic coverage range of a transcript;

Figure 13: Histogram of sampled start sites from the total fragment start site distribution in Figure 10;

Figure 14: Histogram of sampled fragment lengths;

Figure 15: The components (probability subfunctions) of a mixture of Gaussians (dotted lines); solid line: probability distribution function for a transcript;

Figure 16: Convergence of the weight factors alpha for the 1tie-Mix[2] model;

Figure 17: Convergence of the weight factors beta for the 1tie-Mix[2] model;

Figure 18: Final weight factors betas for the 1tie-Mix[2] model;

Figure 19: Final probability distribution function (pdf) for transcript 2 [$p(s(r)|t=2)$] for the 1tie-Mix[2] model;

Figure 20: Convergence of the sum function [$p_{(total)}(s(r))$] for the 1tie-Mix[2] model;

Figure 21: Estimated and true $\alpha_i$ (abundance, ratio of total) for the 1tie-Mix[2] model, the Cufflinks model with alpha$_{(3)}$=0.2, after convergence;

Figure 22: Estimated and true $\alpha_i$ (abundance, ratio of total) for the 1tie-Mix[2] model, the Cufflinks model with alpha$_{(3)}$=0.4, after convergence;

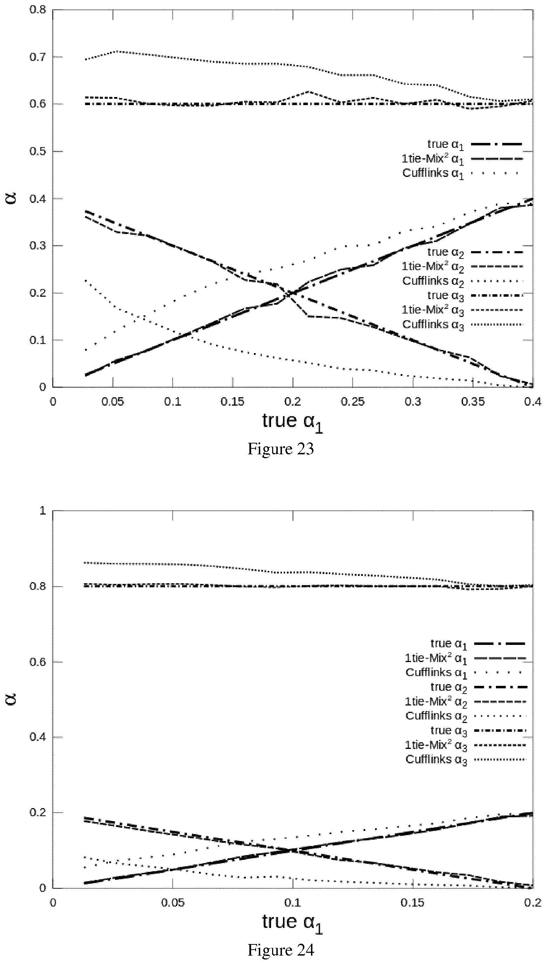

Figure 23: Estimated and true $\alpha_i$ (abundance, ratio of total) for the 1tie-Mix[2] model, the Cufflinks model with alpha$_{(3)}$=0.6, after convergence;

Figure 24: Estimated and true $\alpha_i$ (abundance, ratio of total) for the 1tie-Mix[2] model, the Cufflinks model with alpha$_{(3)}$=0.8, after convergence;

Figure 25: Exon structure of correct and incorrect transcript annotations;

Figure 26: Convergence of the alpha$_i$ for the 5tie-Mix[2] model;

Figure 27: Convergence of the beta$_j$ for the 5tie-Mix[2] model;

Figure 28: Convergence of the mu$_j$ (a parameter for shifting of the probability subfunction j) for the 5tie-Mix[2] model;

Figure 29: Convergence of the sigma$_j$ (a width parameter for the probability subfunction j) for the 5tie-Mix[2] model;

Figure 30: Convergence of the nu$_i$ (a shift or translation factor for the probability distribution function for transcript i) for the 5tie-Mix[2] model;

Figure 31: Convergence of the lambda$_i$ (a scaling factor for the probability distribution function for transcript i) for the 5tie-Mix[2] model;

Figure 32: Convergence of the probability distribution function $p(r|t=i)$ for Transcript 1 and 5tie-Mix[2] model; the solid vertical lines indicate the correct start and end sites of the transcript;

Figure 33: Convergence of the probability distribution function $p(r|t=i)$ for Transcript 2 and 5tie-Mix[2] model; the solid vertical lines indicate the correct start and end sites of the transcript;

Figure 34: Convergence of the probability distribution function $p(r|t=i)$ for Transcript 3 and 5tie-Mix[2] model; the solid vertical lines indicate the correct start and end sites of the transcript;

Figure 35: Convergence of the sum function $p_{(total)}(r)$ for the 5tie-Mix[2] model;

Figure 36: Estimated and true $\alpha_i$ (abundance, ratio of total) for the 5tie-Mix[2] model, the Cufflinks model with alpha$_{(3)}$=0.2, after convergence;

Figure 37: Estimated and true alpha$_i$ (abundance, ratio of total) for the 5tie-Mix$^2$ model, the Cufflinks model with alpha$_{(3)}$=0.4, after convergence;

Figure 38: Estimated and true alpha$_i$ (abundance, ratio of total) for the 5tie-Mix$^2$ model, the Cufflinks model with alpha$_{(3)}$=0.6, after convergence;

Figure 39: Estimated and true alpha$_i$ (abundance, ratio of total) for the 5tie-Mix$^2$ model, the Cufflinks model with alpha$_{(3)}$=0.8, after convergence;

Figure 40: Estimated and true alpha$_i$ for the 5tie-Mix$^2$ model, the 1tie-Mix$^2$ model with alpha$_{(3)}$=0.2, after convergence;

Figure 41: Estimated and true alpha$_i$ for the 5tie-Mix$^2$ model, the 1tie-Mix$^2$ model with alpha$_{(3)}$=0.4, after convergence;

Figure 42: Estimated and true alpha$_i$ for the 5tie-Mix$^2$ model, the 1tie-Mix$^2$ model with alpha$_{(3)}$=0.6, after convergence;

Figure 43: Estimated and true alpha$_i$ for the 5tie-Mix$^2$ model, the 1tie-Mix$^2$ model with alpha$_{(3)}$=0.8, after convergence.

**Examples:**

Example 1: Introduction to NGS methods

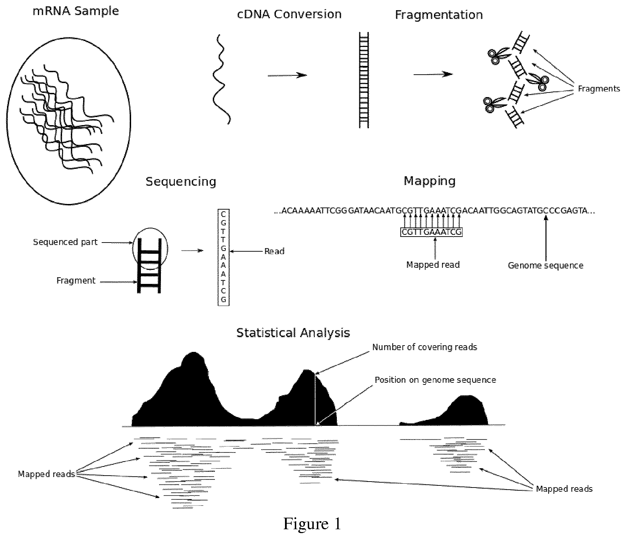

In order to infer which mRNA molecules were present in the original sample, NGS reads are mapped onto a reference genome with known methods, such as the Burrows-Wheeler transform. For each read this gives a set of genetic coordinates, potentially including information about splice sites. The mapping process is visualized in Figure 1. Here the location of the short read that has been produced by the sequencer is identified within the reference genome. This process is repeated for all the reads generated by the sequencer, which results in a large number of short sequences on the genetic axis as indicated by the short straight lines below the filled black curve in Figure 1. The combined statistics of the mapped reads lead to different types of histograms on the genetic axis (i.e. different types of coverage envelope curves). The black filled curve in Figure 1, for instance, depicts the coverage (envelope). At a given position on the genetic axis the value of the curve is the number of reads that cover the position. Other histograms are studied as well like the fragment start site histogram, another type of coverage envelope curve. At each genetic position the value of this histogram is the number of reads starting at this position. The method of the invention does not rely on a special type of histogram but is applicable to any type both on the genetic axis and on the set of fragments in a genetic locus.

Genetic loci such as genes can contain a number of different, possibly overlapping, regions that each generate their own mRNA. Such regions are called transcripts. The histogram in the genetic locus h$_{(locus)}$ (r) is therefore a mixture of the histograms associated with each individual transcript in the locus, i.e.

$$h_{locus}(r) = \sum_{i=1}^{N} h(r|t=i) \qquad (1)$$

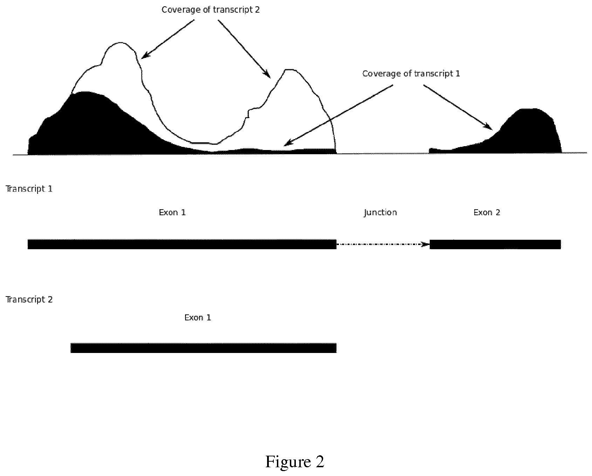

where h(r|t=i) is the histogram associated with transcript t=i and i is the name of a transcript, for instance, Apoe. Figure 2 shows a possible decomposition of the histogram in Figure 1 into the histograms of two transcripts. Transcript 1 extends over the whole length of the genetic locus and starts to the left of transcript 2. In addition, transcript 1 has two exons and one junction. Transcript 2, on the other hand, has a single exon which ends at the end of the first exon of transcript one. The black filled curve in Figure 2 is the histogram of transcript one and the curve on top of it minus the histogram of transcript one is the histogram of transcript 2 (which is unknown from raw fragment sequencing data). The weight $\alpha_i$ of transcript t=i is given by

$$\alpha_i = \frac{\sum_{r \in locus} h(r|t=i)}{\sum_{r \in locus} h_{locus}(r)} \qquad (2)$$

This is the proportion of counts in the total histogram h$_{locus}$(r) that belong to transcript t=i. If h$_{locus}$(r) is, for instance, the histogram of the fragment start sites then $\alpha_i$ is the percentage of fragments that were generated by transcript t=i. In this case, $\alpha_i$ is called the abundance of transcript t=i and is directly correlated to the concentration of transcript t=i within the mRNA sample. In Figure 2 the weight of a transcript is the proportion of the area under the histogram h(r|t=i) to the total area under h$_{locus}$(r). Since the areas of the histograms for both transcripts are roughly the same the weight for each transcript is about 0.5. Thus each transcript has about a 50% chance of covering a position in the genetic locus.

The decomposition of the histogram h$_{locus}$(r) in equation (1) is usually not known and the derivation of the $\alpha_i$ requires elaborate mathematical machinery. In order to make the problem of finding the decomposition (1) mathematically tractable, (1) is reformulated in a probabilistic framework, i.e.

$$p_{locus}(r) = \sum_{i=1}^{N} \alpha_i p(r|t=i) \qquad (3)$$

where $p_{locus}(r)$ is the total probability of observing fragment r in the locus, p(r|t=i) is the probability of observing fragment r in the locus given transcript t=i and $\alpha_i$ is the probability that transcript t=i is observed in the locus. If the probability distributions $p_{locus}(r)$ and p(r|t=i) are derived from the histograms by normalizing them to sum to one then (3) is a direct result of (1) and (2). In order to emphasize the fact that the methods developed in the following are applicable in a general probabilistic setting and not just to the field of next generation sequencing, the subscript "locus" will be replaced by "total", hence

$$p_{(locus)}(r) = p_{(total)}(r) \qquad (4)$$

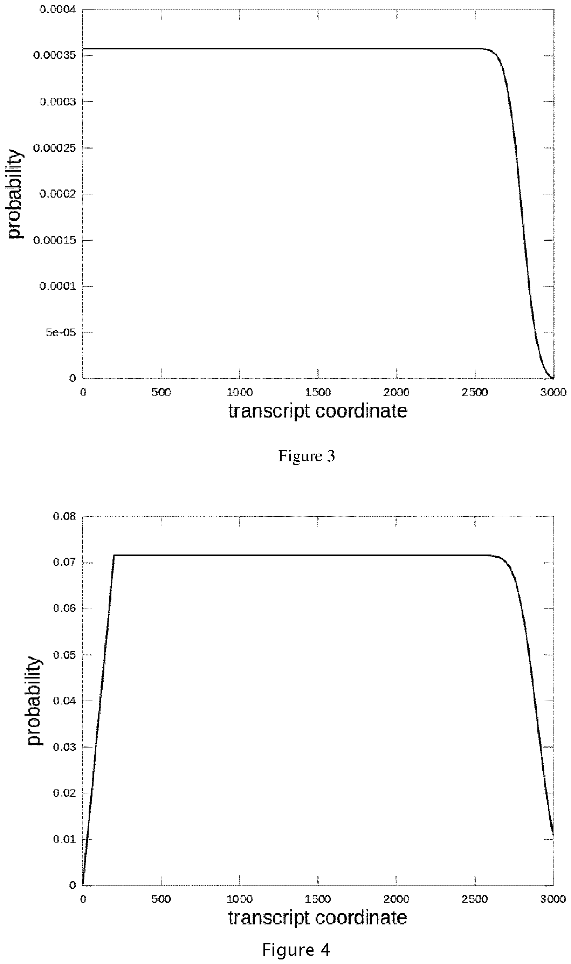

In the estimation of the transcript probabilities $\alpha_i$ the shape of the transcript distributions is of fundamental importance. Current methods such as Cufflinks (Trapnell et al., 2010), however, do not model these distributions accurately. Instead, for the fragment start site distribution Cufflinks uses a model that only depends on the distribution of the fragment lengths. The default model for the fragment lengths in Cufflinks is a Gaussian with mean 200 and a standard deviation of 80. Together with the other assumptions in Cufflinks this implies that for a read length of 200 bp's and a transcript of 3000 bp's the fragment start site distribution and the coverage of the transcript are modeled by the functions shown in Figures 3 and 4.

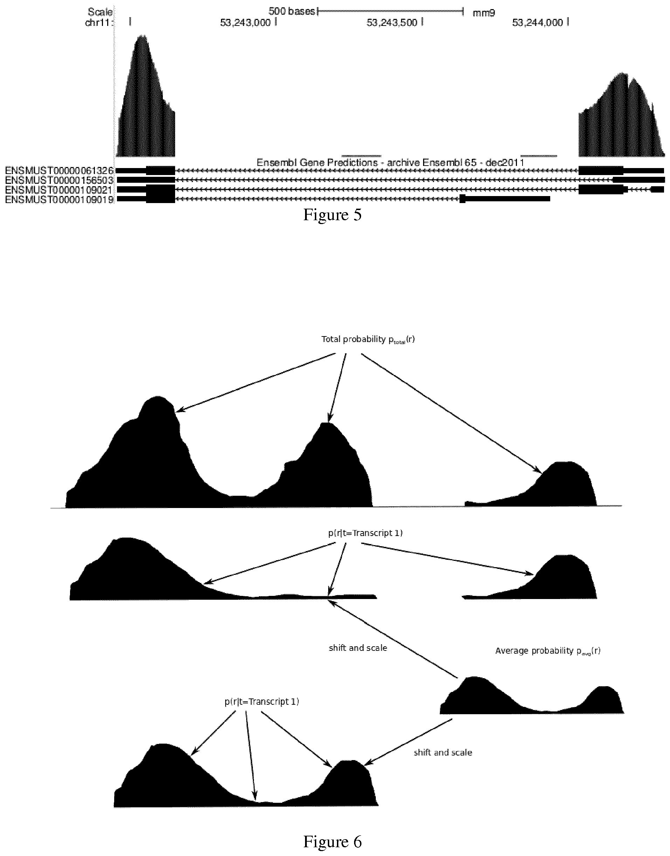

In comparison, Figure 5 shows the coverage of the Square library prep, which deviates significantly from the distribution assumed by Cufflinks in Figure 4. Biases in coverage towards the 5' and/or 3' end of a transcript are a common feature among current NGS libraries and the assumptions of Cufflinks are therefore invalid. As a consequence, the transcript abundances and concentrations estimated by Cufflinks can be substantially different from their true values. The method described here estimates simultaneously the transcript distributions and their probabilities and is therefore considerably more accurate in the estimation of transcript abundances.

The distributions observed in a genetic location often exhibit visual clues as to which transcripts are contained in the genetic location and their probability. Consider, again, the example in Figure 2. If it is known that the average distribution of a transcript has a shape similar to the one in Figure 6 and that the transcript probabilities p(r|t=i) are approximately derived from $p_{avg}(r)$ by scaling and shifting, then the task of finding the components of the superposition in Figure 2 can be solved by looking for shapes in the total probability distribution $p_{total}(r)$ that are similar to the shapes of the average distribution $p_{avg}(r)$. This process is visualized in Figure 6, which indicates that the best fit to $p_{total}(r)$ by a superposition of scaled and shifted versions of $p_{avg}(r)$ leads to a superposition similar to that in Figure 2 and thus to a correct estimation of the transcript probabilities $\alpha_i$.

Since the p(r|t=i) are scaled and shifted versions of $p_{avg}(r)$, they are given by

$$p(r|t=i) = p_{avg}(\lambda_i r - \nu_i) \qquad (5)$$

where $\lambda_i$ and $\nu_i$ are the scale and shift parameters of transcript t=i. This means that a transcript t=i only has two parameters $\lambda_i$ and $\nu_i$ that are unique to the transcript. All other parameters, i.e. the parameters of $p_{avg}(r)$, are tied between the different p(r|t=i). This highlights a central idea of the method described in the following that structures that are common to different transcripts, such as $p_{avg}(r)$, can be estimated by appropriately tying the parameters of the p(r|t=i). In the following the p(r|t=i) will be modeled by a mixture of functions and the decomposition of $p_{total}(r)$ in (3) is therefore a mixture of mixtures of functions. In order to increase readability this model will be referred to as the inventive model. The following sections give a general introduction to the inventive model, also called the Mix$^2$ model, and discuss several of its variants. In addition, experiments show that the inventive model, the Mix$^2$ model, can reliably estimate the probability distributions p(r|t=i) and produces estimates for the $\alpha_i$ that are considerably more accurate than those of the Cufflinks model.

Example 2: Coordinate transformations

2.1. Positions in genome and transcript coordinates

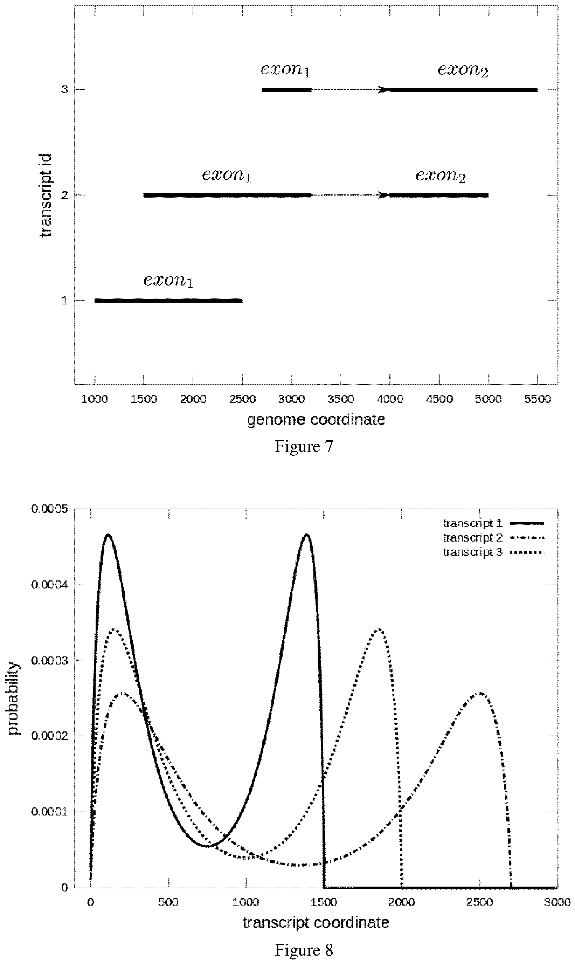

The genetic axis is the sequence of base pairs that have been sequenced for an organism, which usually starts at zero or one and can reach up to several hundred million base pairs long, depending on the complexity of the organism. In addition, the genetic axis is usually subdivided into chromosomes or contigs. The genetic axis is visualized at the top of Figure 5 which indicates that this graphic represents a selection of a genome on chromosome 11 approximately between base pair 53,242,500 and 53,244,200. A transcript is usually defined as a sequence of exons (exon$_1$,...,exon$_N$) on the genetic axis, where the i-th exon is an interval [s(exon$_i$),...,e(exon$_i$)] on the genetic axis which starts at s(exon$_i$) and ends at e(exon$_i$). The gap between two successive exons [e(exon$_i$)+1,s(exon$_{i+1}$)-1] is called an intron and the connection from the last nucleotide preceding an intron to the first nucleotide following an intron is called a junction. Three examples of transcripts are shown in Figure 7. The x-axis on this figure gives the genetic coordinates, which range from 1000 to 5500, while the y-axis gives the transcript id. Thus, transcript 1 consists of a single exon which starts at position 1000 and ends at position 2500. Transcript 2, on the other hand, is defined by the exon sequence ([1500,3200],[4000,5000]), whereas transcript 3 is defined by the exon sequence ([2700,3200], [4000,5500]). Hence, transcript 2 and 3 have the same junction which is indicated in Figure 7 by a dashed arrow. In the following, the length of a transcript will be denoted by 1(t). Thus 1(Transcript 1)=1501, 1(Transcript 2)=2702 and 1(Transcript 3)=2002. The exon sequence and the length of the transcripts in Figure 7 is also summarized in Table 1.

Table 1: Annotation of transcripts in Figure 7

|  | Exon 1 | Exon 2 | Length |
|---|---|---|---|
|  |  |  |  |
| Transcript 1 | 1000,2500 | NA | 1501 |
| Transcript 2 | 1500,3200 | 4000,5000 | 2702 |
| Transcript 3 | 2700,3200 | 4000,5500 | 2002 |

Points on the genetic axis that lie within an exon of a transcript can also be referenced within the coordinate system of this transcript. A transcript coordinate is the distance from the start of the transcript minus the length of the preceding introns and is therefore a number between 1 and 1(t). Thus for a position P in exon e$_i$ of a transcript the transcript and genome coordinates P$_{trans}$ can be converted as follows,

$$P_{trans} = P_{genome} - s(exon_1) + 1 - \sum_{j=1}^{i} s(exon_j) - e(exon_{j-1}) - 1 \qquad (6)$$

and

$$P_{genome} = P_{trans} + s(exon_1) - 1 + \sum_{j=1}^{i} s(exon_j) - e(exon_{j-1}) - 1 \qquad (7)$$

Consider, for instance, the position 4500 in genetic coordinates in Figure 7. This position lies within exon$_2$ of both the second and third transcript. For transcript two, the transcript coordinate of this position is 4500-1500+1-801=2200, while for transcript three the transcript coordinate is 4500-2700+1-801=1000. Since the position 4500 does not lie within an exon of transcript one it is not possible to convert this position into transcript coordinates for transcript 1. A position on the genetic axis that lies within an exon of a transcript will be called compatible with the transcript. The transform of a position in genetic (e.g. genomic) coordinates that is compatible with a transcript into the coordinates of the transcript will be denoted by T. The transcript that T is associated with will become clear from the context. Probability distributions in transcript coordinates p$_{trans}$(P$_{trans}$|t=i) can be converted into probability distributions in genetic or genome coordinates as follows

$$p_{genome}\left(P_{genome}|t=i\right)=\begin{cases} p_{trans}\left(T\left(P_{genome}\right)|t=i\right) & \text{if } P_{genome} \text{ is compatible with transcript } t=i \\ 0 & \text{if } P_{genome} \text{ is incompatible with transcript } t=i \end{cases}$$

$$(8)$$

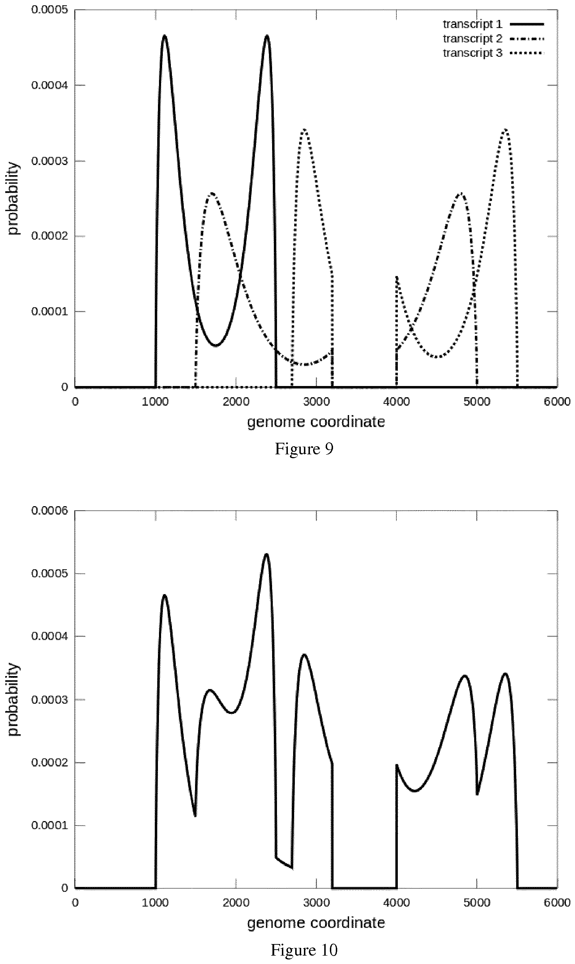

In order to simplify notation, the "genome" and "trans" subscripts will be dropped in the following whenever convenient. Figures 8 and 9 show the relationship between probability distributions in transcript coordinates and genome coordinates. Figure 8 shows a scaled probability distribution for each of the three transcripts in Figure 7. Anticipating their use as components of a mixture each probability distribution has been multiplied with a factor of 1/3. The solid line belongs to transcript one which is the shortest of the three transcripts and has a length of 1501 base pairs. Transcript two and three, on the other hand, are 2002 and 2702 base pairs long and their probability distributions in transcript coordinates therefore range from 1 to 2002 and 2702, respectively.

Figure 9 shows the transcript probability distributions in genome coordinates. Here the different start sites and the junctions of the transcript have been taken into account.

Taking the sum of the transcript probabilities in genome coordinates yields the total probability distribution $p_{total}(r)$ for positions in the entire genetic locus of interest, which is shown in Figure 10. The curve in Figure 10 is similar to a smoothed version of the distributions that are derived in a genetic locus from NGS data. In later sections, distributions like the one in Figure 10 will be used to generate samples in the genetic locus which are interpreted as fragment start sites. These samples will then be used to train the parameters of the probability density functions of the inventive model and the resulting $p(r|t=i)$ and $p_{total}(r)$ will be shown to be accurate estimates of the curves in Figures 9 and 10, which subsequently yield the abundance as weight factor $\alpha_i$.

2.2 Fragments in genome and transcript coordinates

A fragment is a continuous sequence within a transcript. Similar to a transcript, a fragment r therefore consists of a sequence of intervals on the genetic axis, r=(rint$_1$,...,rint$_K$), where rint$_i$=[s(rint$_i$),e(rint$_i$)] is the i-th interval with start s(rint$_i$) and end e(rint$_i$). A fragment r is compatible with a transcript if its start and end are located within exons of the transcript and the gaps between adjacent intervals [e(rint$_i$)+1,s(rint$_{i+1}$)-1] are introns of the transcript, i.e.

$$\mathtt{s(int_1) \in exon_i} \hspace{4cm} (9)$$

$$\mathtt{e(int_K) \in exon_k} \hspace{4cm} (10)$$

for some i ≤ k, and

$$[e(int_k)+1, s(int_{k+1})-1] \in \{[e(exon_i)+1, s(exon_{i+1})-1] : i = 1, \ldots, N-1\} \forall k = 1, \ldots, K-1$$

$$(11)$$

If a fragment r is compatible with a transcript it can be converted into the interval [T(s(rint$_1$)),T(e(rint$_K$))] in transcript coordinates. The gaps between intervals of the fragment are therefore removed in the transformation to transcript coordinates. As in the previous section, the transform of a fragment r to transcript coordinates will be denoted by T. Consider, for instance, the fragment ([2000,3000], [4000,4500]) and the three transcripts in Figure 7. The start and end of this fragment lie in the first and second exon of transcript two, respectively. At the same time the gap [3001,3999] of the fragment coincides with the intron of transcript two. Thus the fragment is compatible with transcript two and is transformed into the interval [501,2200] in transcript coordinates. On the other hand, for transcript one and transcript three either the start or end of the fragment do not lie within one of its exons and the fragment is therefore not compatible with either of these transcripts.

Probability distributions on fragments in transcript coordinates $p_{trans}(r_{trans}|t=i)$ can therefore be converted into probability distributions on fragments in genomic coordinates as follows.

$$p_{genome}(r_{genome}|t=i) \quad = \quad \begin{cases} p_{trans}\left(T\left(r_{genome}\right)|t=i\right) & r_{genome}\text{ is compatible with } t{=}i \\ 0 & r_{genome}\text{ is not compatible with } t{=}i \end{cases} \qquad (12)$$

As before, the "genome" and "trans" subscripts will be dropped whenever convenient. Instead of using its start and end, an interval can also be represented by its start s(r) and length 1(r). This allows the following factorizations of probability distributions in transcript coordinates.

$$p_{trans}(r|t=i) = p_{trans}(s(r)|t=i,l(r))p_{trans}(l(r)|t=i) \qquad (13)$$

and

$$p_{trans}(r|t=i) = p_{trans}(l(r)|t=i,s(r))p_{trans}(s(r)|t=i) \qquad (14)$$

These factorizations are convenient for NGS data as the global distribution of the fragment lengths can usually be inferred from bioanalyzer traces of the library. The factorization in (13) is used by Cufflinks, which additionally assumes that a fragment of a given length can be placed anywhere on the transcript with equal probability. In contrast, in the experiments in section 4 the Mix$^2$ model uses the factorization in (14) which allows a more efficient estimation of the transcript specific variability of the fragment start sites.

Example 3: Estimation of transcript probabilities and transcript specific probability distributions

The model that is described in the following uses mixtures of mixtures of functions and will therefore be called the Mix$^2$ model.

3.1 Mathematical foundations of the Mix$^2$ model

In the following, r can represent both a fragment and a position. However, for convenience, r will always be referred to as a fragment. The probability of observing a particular fragment r in the genetic locus $p_{total}(r)$ is a sum of the probabilities of observing the fragment for a transcript weighted by the probability that the transcript generates a fragment. Hence the $p_{total}(r)$ is given by the following mixture of probability distributions.

$$p_{total}(r) = \sum_{i=1}^{N} \alpha_i p(r|t=i) \qquad (15)$$

As described in section 2, if r is compatible with t = i then $p(r|t=i) = p_{trans}(T(r)|t=i)$ and $p(r|t=i) = 0$ otherwise. The method assumes that the probability distributions $p_{trans}(r|t=i)$ are mixtures, i.e.

$$p_{trans}(r|t=i) = \sum_{j=1}^{M_i} \beta_{i,j} p_{trans}(r|t=i,b=j) \qquad (16)$$

Here $M_i$ is the number of mixture components and the $p_{trans}(r|t{=}i,b{=}j)$ are probability distributions. The $\beta_{i,j} \geq 0$ are weights which sum to one, i.e.

$$\sum_{j=1}^{M_i} \beta_{i,j} = 1 \qquad (17)$$

As before, the variable t is meant to represent a transcript, whereas the newly introduced variable b is meant to represent a "building block", also referred to as probability subfunction in general. In this model it is possible to calculate the posterior probability that transcript t=i and block b=j were observed given a fragment r. This posterior probability is given by

$$p(t=i, b=j|r) = \frac{\alpha_i \beta_{i,j} p(r|t=i, b=j)}{\sum_{i=1}^{N} \sum_{j=1}^{M_i} \alpha_i \beta_{i,j} p(r|t=i, b=j)} \qquad (18)$$

Note that $p(r|t=i, b=j) = 0$ and hence $p(t=i, b=j|r) = 0$ if fragment $r$ is incompatible with transcript $t=i$. The posterior probabilities for $t$ and $b$ are given by

$$p(t=i|r) = \frac{\alpha_i p(r|t=i)}{\sum_{i=1}^{N} \alpha_i p(r|t=i)} \qquad (19)$$

and

$$p(b=j|r) = \frac{\sum_{i=1}^{N} \alpha_i \beta_{i,j} p(r|t=i, b=j)}{\sum_{i=1}^{N} \sum_{j=1}^{M_i} \alpha_i \beta_{i,j} p(r|t=i, b=j)} \qquad (20)$$

The model assumes furthermore that some of the parameters of $p_{total}(r)$ are tied between different transcripts (probability distribution functions) and blocks (probability subfunctions). This can include both the mixture weights $\beta_{i,j}$ as well as any parameters $\Theta$ that determine the set of probability subfunctions of the probability distributions $p_{trans}(r|t=i, b=j)$, i=1,...,N, j=1,...,M. The tying might be applied to all transcripts and blocks combined or within groups of transcripts and blocks. Since tying the parameters of probability distributions $p_{trans}(r|t=i)$ implies certain similarities between the $p_{trans}(r|t=i)$, parameter tying should only be applied to transcripts which exhibit the kind of similarity implied by the type of parameter tying. If, for instance, the parameter tying implies that the $p_{trans}(r|t=i)$ are derived from a single $p_{avg}(r)$ by scaling and shifting, as has been suggested in the introduction, then the parameter tying should only be applied to transcripts for which the $p_{trans}(r|t=i)$ are scaled and shifted versions of one another. For some library preps the latter is only valid for transcripts with lengths in a certain range. Transcripts whose lengths lie within different ranges should therefore not apply a parameter tying which requires the $p(r|t=i)$ to be scaled and shifted versions of one another.

For a data set R of fragments r in a genetic region, the transcript specific distributions $p(r|t=i)$ and the transcript probabilities $\alpha_i$ of the Mix$^2$ model are estimated by maximizing the likelihood of the data set R under the model $p_{total}(r)$. The likelihood function of the parameters $\alpha_i, \beta_{i,j}, \Theta$ is therefore given by

$$\mathcal{L}\left((\alpha_i, \beta_{i,j}, \Theta)|R\right) = \sum_{r \in R} \log \left( \sum_{i=1}^{N} \alpha_i \sum_{j=1}^{M_i} \beta_{i,j} p(r|t=i, b=j) \right) \qquad (21)$$

The maximization of (21) is a constrained non-linear optimization problem for which approximate solutions can be found with a number of different optimization methods. The optimization method used in the following is the expectation maximization (EM) algorithm. The EM algorithm is an iterative procedure that finds a local maximum of the likelihood function. In order to obtain a local maximum close to the global optimum it maybe necessary to try the EM algorithm several times with different initializations for the model parameters. The result that yields the highest likelihood is then chosen as the solution to the optimization problem. The EM algorithm can be used to estimate the $\alpha_i$ without any assumptions about the form of the $p_{trans}(r|t=i)$ or the tying of their parameters. The EM update formula for the $\alpha_i$, which is also used by Cufflinks, is given by

$$\alpha_i^{(n+1)} = \frac{1}{|R|} \sum_{r \in R} p^{(n)}(t=i|r) \qquad (22)$$

Here |R| is the number of fragments in data set R, $\alpha_i^{(n+1)}$ is the estimate for $\alpha_i$ after the n+1-th iteration and $p^{(n)}(t=i|r)$ is the posterior probability of observing transcript t=i given fragment r, estimated with the parameters from the n-th iteration. Since in this case the optimization problem is concave, it is not necessary to repeat the EM algorithm with different initializations. For concave problems there is a single global optimum to which the EM algorithm converges always. The model parameters are estimated by optimizing the fit of the model $p_{total}(r)$ to the observed data R. However, the ultimate goal is to find a good fit to the $p_{trans}(r|t=i)$ and therefore a good estimate of the transcript probabilities $\alpha_i$. The optimization of $p_{total}(r)$ per se does not imply a good estimate of the $\alpha_i$. Only if the parameters of the $p_{trans}(r|t=i)$ are tied appropriately will the optimization of $p_{total}(r)$ result in a good estimate of the transcript probabilities $\alpha_i$. The next section introduces several variants of the Mix$^2$ model which use different tying strategies. Two of those variants will be studied in section 4 where they will be shown to yield considerably more accurate estimates for the $\alpha_i$ than the Cufflinks model, for the case of scaled and shifted transcript probability distributions p(r|t=i).

3.2 Variants of the Mix$^2$ model

The mathematical foundations of the Mix$^2$ model in the last section are fairly general. This section discusses a number of concrete realizations of the Mix$^2$ model and highlights their various advantages and limitations.

3.2.1 Tying within a single group of parameters

The simplest Mix$^2$ model discussed in this section ties only the weights $\beta_{i,j}$ for i=1,...,N between different transcripts. Thus the set of parameters of this model is $\{\alpha_i, \beta_j: i=1,...,N, j=1,...,M\}$ and $p_{trans}(r|t=i)$ is given by

$$p_{trans}(r|t=i) = \sum_{j=1}^{M} \beta_j p_{trans}(r|t=i, b=j) \qquad (23)$$

Since this Mix$^2$ model only ties parameters within a single group of parameters, namely the $\beta_j$, this model will be called the 1tie-Mix$^2$ model in the following. The probability of observing a fragment r under the 1tie-Mix$^2$ model is given by

$$p_{total}(r) = \sum_{i=1}^{N} \alpha_i \sum_{j=1}^{M} \beta_j p_{trans}(T(r)|t=i, b=j) \qquad (24)$$

In equation (24) the order of the sums can be interchanged which yields

$$p_{total}(r) = \sum_{j=1}^{M} \beta_j p(r|b=j) \qquad (25)$$

where

$$p(r|b=j) = \sum_{i=1}^{N} \alpha_i p_{trans}(T(r)|t=i, b=j) \qquad (26)$$

As previously, summands in (26) are zero if fragment r is not compatible with transcript t=i. Since p(r|b=j) is a probability distribution equation (25) shows that, given the $\alpha_i$, the problem of estimating the $\beta_j$ is conceptually the same as the estimation of the $\alpha_i$. Thus the EM algorithm can be applied and gives the following update formula for $\beta_j$

$$\beta_j^{(n+1)} = \frac{1}{|R|} \sum_{r \in R} p^{(n)}(b=j|r) \qquad (27)$$

This implies that both the $\alpha_i$ and the $\beta_j$ of the 1tie-Mix$^2$ model can be trained simultaneously by iterative application of the formulas (22) and (27). In principle, the EM algorithm for the 1tie-Mix$^2$ model can converge to different solutions depending on the initialization of the $\alpha_i$ and $\beta_j$ since, in comparison to the Cufflinks model, the likelihood function of the 1tie-Mix$^2$ model is not concave. In the experiments in Section 4, however, a single initialization of the $\alpha_i$ and $\beta_j$ was sufficient in order to converge to a satisfactory result. The probability distributions $p_{trans}(r|t=i,b=j)$ for the 1tie-Mix$^2$ model have to be chosen to fit the data. Thus previous knowledge about the structure of the data is necessary. If the observed $p_{trans}(r|t=i)$ are scaled versions of one another for different t=i, then the $p_{trans}(r|t=i,b=j)$ should be scaled versions for different t=i as well. In addition, for the same b=j but different t=i the $p_{trans}(r|t=i,b=j)$ have to lie within regions of the transcript coordinate system which are regulated by the same scaling factor $\beta_j$. The latter can be achieved if the correct start and length of the transcripts are known. In this case the $p_{trans}(r|t=i,b=j)$ can be placed at equidistant positions along the transcript coordinate system of t=i. If the correct start and length are not known it is not immediately clear how to position the $p_{trans}(r|t=i,b=j)$. The model in the next section avoids this restriction by learning the transcript start and length from the data.

3.2.2 Tying within 5 groups of parameters

The model in the last section relies on the knowledge of the correct start sites and lengths of the transcripts. This section discusses a model that positions and scales the $p_{trans}(r|t=i,b=j)$ automatically. Since this model ties parameters within 5 groups of parameters it will be called the 5tie-Mix$^2$ model in the following. The 5tie-Mix$^2$ model uses Gaussians for the distributions $p_{trans}(r|t=i,b=j)$, whose internal parameters, namely their means $\mu_{i,j}$ and standard deviations $\sigma_{i,j}$ are derived from the set of parameters $\Theta = \left\{ \widehat{\mu_{i,j}}, \widehat{\sigma_{i,j}}, \widehat{\nu_{i,j}}, \widehat{\lambda_{i,j}} : i = 1,..., N, j = 1,..., M \right\}$. In particular the $\mu_{i,j}$ and $\sigma_{i,j}$ are given by

$$\mu_{i,j} = \widehat{\lambda_{i,j}}\widehat{\mu_{i,j}} + \widehat{\nu_{i,j}} \tag{28}$$

$$\sigma_{i,j} = \widehat{\lambda_{i,j}}\widehat{\sigma_{i,j}} \tag{29}$$

The $\widehat{\mu_{i,j}}$ and $\widehat{\sigma_{i,j}}$ are tied between different transcripts, hence $\widehat{\mu_{i,j}} = \mu_j$ and $\widehat{\sigma_{i,j}} = \sigma_j$, whereas the $\widehat{\lambda_{i,j}}$ and $\widehat{\nu_{i,j}}$ are tied between different blocks, hence $\widehat{\lambda_{i,j}} = \lambda_i$ and $\widehat{\nu_{i,j}} = \nu_i$. As a result equations (28) and (29) reduce to

$$\mu_{i,j} = \lambda_i\mu_j + \nu_i \tag{30}$$

$$\sigma_{i,j} = \lambda_i\sigma_j \tag{31}$$

The pdf for transcript $t = i$ and block $b = j$ are therefore given by

$$p(r|t=i,b=j) = \frac{1}{\lambda_i\sigma_j\sqrt{2\pi}}e^{-\frac{\left(r-\lambda_i\mu_j-\nu_i\right)^2}{2\left(\lambda_i\sigma_j\right)^2}} \tag{32}$$

As before, the $\beta_{i,j}$ are tied for block j between the transcripts $i = 1,..., N$ and hence $\beta_{i,j} = \beta_j$. The probability distribution $\sum_{j=1}^{M}\beta_j g_{\mu_j,\sigma_j}(r)$, where $g_{\mu_j,\sigma_j}(r)$ is a Gaussian with mean $\mu_j$ and standard deviation $\sigma_j$, can be interpreted as the average pdf $p_{avg}(r)$ of the transcripts in the locus which is shifted by $\nu_i$ and scaled by $\lambda_i$ to obtain the pdf $p(r|t=i)$ for transcript $t = i$. Without restricting the power of the model it is possible to set $\lambda_i = 1$ and $\nu_i = 0$ for a transcript of choice. The pdf in equation (32) is not a probability distribution in the coordinate system of transcript $t = i$ but in the real continuous space. Hence the resulting pdf's $p(r|t = i)$ and $p_{total}(r)$ are also continuous pdf's. In order to emphasize this point, these probability distributions will be denoted by $p_{\mathbb{R}}(r|t = i, b = j)$, $p_{\mathbb{R}}(r|t = i)$ and $p_{total,\mathbb{R}}(r)$ in the following and the weight of $p_{\mathbb{R}}(r|t = i)$ will be denoted by $\alpha_{i,\mathbb{R}}$. From these continuous probability distributions, the $p_{total}(r)$ and $p_{trans}(r|t = i)$ in genome and transcript coordinates can be calculated by restricting the $p_{trans,\mathbb{R}}(r|t = i)$ to the set of possible fragments $F(t)$ for transcript $t = i$ and normalizing their values to sum to one, i.e.

$$p_{total}(r) = \frac{p_{total,\mathbb{R}}(r)}{\sum_{r \in \cup F(t)} p_{total,\mathbb{R}}(r)} \tag{33}$$

$$p_{trans}(r|t = i) = \frac{p_{trans,\mathbb{R}}(r|t = i)}{\sum_{r \in F(t)} p_{trans,\mathbb{R}}(r|t = i)} \tag{34}$$

where the union in the sum of the denominator in (33) extends over all the transcripts in the locus. The $\alpha_i$ can then be derived from the $\alpha_{i,\mathbb{R}}$ as follows

$$\alpha_i = \frac{\alpha_{i,\mathbb{R}} \sum_{r \in F(t)} p_{trans,\mathbb{R}}(r|t = i)}{\sum_{j=1}^{N} \alpha_{j,\mathbb{R}} \sum_{r \in F(t)} p_{trans,\mathbb{R}}(r|t = j)} \tag{35}$$

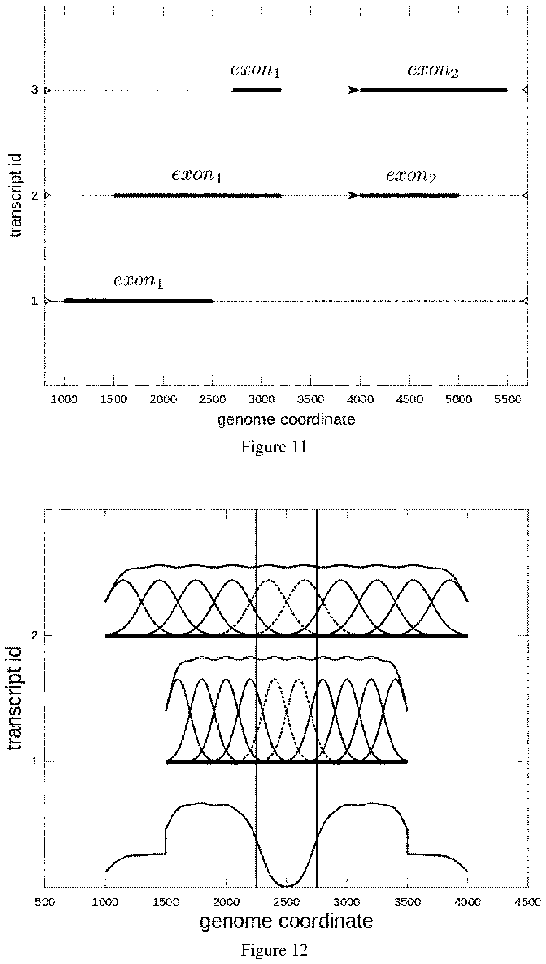

Equation (35) amounts to a rescaling of the continuous model weights alpha$_{(i,R)}$. The effect of the rescaling is not very pronounced for slowly varying probability distributions $p_{trans}(r|t=i)$ and for genetic loci in which all the transcripts have a sensible length. Thus in the experiments described later the effect of the rescaling formula is negligible. In situations where the locus and the $p_{trans}(r|t=i)$ conform to these requirements the alpha$_{(i,R)}$ can therefore be directly used as the transcript probabilities $\alpha_i$, which will be done in Section 4. The $\lambda_i$ are scaling parameters that account for the different lengths of the transcripts, whereas the $\nu_i$ account for the different relative offsets of the transcript start sites from the transcript annotation. Since these two parameters can correct for incorrect start sites and lengths in the transcript annotation it is possible to remove the definition of start and end all together from the transcript annotations. For instance, the start sites of all the transcripts can be set to the same value, which is smaller than all the start sites of the transcripts in the locus. Likewise, the end sites of all the transcripts can be set to the same value, which is larger than all the end sites of the transcripts in the locus. This extension of the transcript annotations is illustrated in Figure 11. Extending the transcript annotations has the effect that fragments that were previously invalid for a transcript due to their incompatibility with the transcript start or end become now valid for this transcript. In Figure 11, for instance, all the fragments that are valid for transcript two are also valid for transcript three. In addition, all the fragments that do not span or enter the intron are valid for all three transcripts.

The parameters of the model in this section can be efficiently trained with the EM algorithm. The EM update formulas for the means, variances and offset parameters are given below.

$$\mu_j^{(n+1)} = \frac{\sum_{i=1}^{N} \sum_{r \in R} p^{(n)}(t = i, b = j|r) \frac{r - \nu_i^{(n)}}{\lambda_i^{(n)}}}{\sum_{i=1}^{N} \sum_{r \in R} p^{(n)}(t = i, b = j|r)} \tag{36}$$

$$\sigma_j^{(n+1)} = \left( \frac{\sum_{i=1}^{N} \sum_{r \in R} p^{(n)}(t = i, b = j|r) \left( \frac{r - \nu_i^{(n)}}{\lambda_i^{(n)}} - \mu_j^{(n)} \right)^2}{\sum_{i=1}^{N} \sum_{r \in R} p^{(n)}(t = i, b = j|r)} \right)^{\frac{1}{2}} \tag{37}$$

$$\nu_i^{(n+1)} = \frac{\sum_{j=1}^{M} \sum_{r \in R} p^{(n)}(t=i, b=j|r) \frac{r - \lambda_i^{(n)} \mu_j^{(n)}}{\left(\sigma_j^{(n)}\right)^2}}{\sum_{i=1}^{N} \sum_{r \in R} p^{(n)}(t=i, b=j|r) \frac{1}{\left(\sigma_j^{(n)}\right)^2}} \tag{38}$$

Calculating the derivative of the auxiliary function of the EM algorithm with respect to the scale parameter $\lambda_i$ leads to the following quadratic equation.

$$\left(\lambda_i^{(n+1)}\right)^2 \sum_{j=1}^{M} \sum_{r \in R} p^{(n)}(t=i, b=j|r)$$

$$+ \lambda_i^{(n+1)} \sum_{j=1}^{M} \sum_{r \in R} p^{(n)}(t=i, b=j|r) \frac{\mu_j \left(r - \nu_i^{(n)}\right)}{\left(\sigma_j^{(n)}\right)^2}$$

$$- \sum_{j=1}^{M} \sum_{r \in R} p^{(n)}(t=i, b=j|r) \frac{\left(r - \nu_i^{(n)}\right)^2}{\left(\sigma_j^{(n)}\right)^2} \tag{39}$$

$$= 0$$

In most practical circumstances equation (39) has one positive and one negative solution. Since $\lambda_i$ is a scale parameter it has to be positive and therefore only the positive solution of (39) is of interest. The experiments in section 4 show that the 5tie-Mix$^2$ model can indeed correct imprecise transcript annotations and yields in this case much more accurate estimates for the $\alpha_i$ than models which rely on correct transcript annotations.

3.2.3 Tying within 2 groups of parameters

As the previous models, the model in this section ties the $\beta_{i,j}$ across transcripts for block j and therefore $\beta_{i,j} = \beta_j$. In addition the model in this section ties the $\widehat{\sigma_{i,j}}$ from the 5tie-Mix$^2$ model but not across transcripts for a given block but more generally across sets of pairs of $(i,j)$, $i = 1,..., N$, $j = 1,...,M$. For instance, if $N \geq 2$, $M \geq 3$ then a possible set is $L = \{(1,1), (1,2), (2,3)\}$ and hence $\widehat{\sigma_{1,1}} = \widehat{\sigma_{1,2}} = \widehat{\sigma_{2,3}} = \sigma_L$. Since the model in this section only tics parameters within two groups it will be called the 2tie-Mix$^2$ model. Thus for the 2tie-Mix$^2$ model

$$\sigma_{i,j} = \lambda_i \sigma_L \ (i,j) \in L \tag{40}$$

The motivation for the 2tie-Mix$^2$ model is the following. Consider the situation depicted in Figure 12. In this figure two transcripts without any junctions lie in the region starting at 1000 bp's and ending at 4000 bp's. The second transcript spans the complete region, while the first transcript starts at position 1500 and ends at position 3500. The pdf $p(r|t=i)$ of each transcript is supposed to have the shape of the two curves above the Gaussians in Figure 12. However, the total pdf $p_{total}(r)$ which is depicted at the bottom of Figure 12 is not exactly the sum of the $p(r|t=i)$ for the two transcripts. Instead, $p_{total}(r)$ has a dip in the region between 2250 bp's and 2750 bp's. This is a common feature of NGS sequencing where the nucleotide sequence within certain regions can affect the generation of fragments for all the transcripts intersecting this region. This can be the result of the inability of the sequencing primers, which initiate the sequencing, to bind to certain sequences. In the example in Figure 12, for instance, the sequences within the region of the dip might be less favored by the sequencing primers and the total pdf $p_{total}(r)$ in this region is therefore markedly reduced. The solution to this problem is not the tying of the $\beta_j$ which might be shared by other transcripts not intersecting the region of the dip but the tying of the $\widehat{\sigma_{i,j}}$ that belong to blocks $p(r|t= i, b = j)$ which have an intersection with the region. In Figure 12 these blocks are indicated by the dashed lines. If the $\widehat{\sigma_{i,j}}$ of all these blocks are set to very large values, then the Gaussians $p(r|t = i, b = j)$ spread over the whole continuous x-axis and contribute hardly anything to the total pdf $p_{total}(r)$ in the genomic locus. If the regions where a dip in $p_{total}(r)$ can occur are not known in advance, all the $\widehat{\sigma_{i,j}}$ that belong to blocks which are close to each other can be tied. In the present example the $\widehat{\sigma_{i,j}}$ of two adjacent blocks for transcript one might be tied with the $\widehat{\sigma_{i,j}}$ of the block of transcript two lying above them, e.g. $L = \{(1,1),(1,2),(2,3)\}$ and hence, as at the beginning of this section, $\widehat{\sigma_{1,1}} = \widehat{\sigma_{1,2}} = \widehat{\sigma_{2,3}} = \sigma_L$. The EM update formula for the $\sigma_L$ can be derived from equation (37) and is given by

$$\sigma_L^{(n+1)} = \left( \frac{\sum_{(i,j)\in L} \sum_{r\in R} p^{(n)}(t = i, b = j|r) \left( \frac{r-\nu_i^{(n)}}{\lambda_i^{(n)}} - \mu_j^{(n)} \right)^2}{\sum_{(i,j)\in L} \sum_{r\in R} p^{(n)}(t = i, b = j|r)} \right)^{\frac{1}{2}} \tag{41}$$

The EM update formula for the other parameters of the model is the same as in the previous section. In the situation in Figure 12 updating the $\beta_j$ is sensible. Updating the other parameters might destroy the spacial association of the $p(r|t = i, b = j)$ for $(i,j) \in L$ and is therefore potentially harmful.

3.2.4 Tying within 6 groups of parameters

A straight-forward extension to the 5tie-Mix$^2$ model is given by the introduction of another parameter $\widehat{\kappa_{i,j}}$ which scales the $\widehat{\sigma_{i,j}}$. In particular

$$\sigma_{i,j} = \widehat{\kappa_{i,j}}\widehat{\sigma_{i,j}} \tag{42}$$

Thus, in comparison to the 5tie-Mix$^2$ model, the model in this section scales $\widehat{\mu_{i,j}}$ and $\widehat{\sigma_{i,j}}$ independently and since parameters are tied within 6 groups of parameters it is called the 6tic-Mix$^2$ model. For the 6tie-Mix$^2$ model the $\widehat{\kappa_{i,j}}$ are tied for the same transcript between different blocks, hence $\widehat{\kappa_{i,j}} = \kappa_i$. In the 6tie-Mix$^2$ model the $\lambda_i$ and $\kappa_i$ play a role similar to the means $\mu_j$ and standard deviations $\sigma_j$ and can be trained with the following formulas, which have a similar structure as equations (36) and (37)

$$\lambda_i^{(n+1)} = \frac{\sum_{j=1}^{M} \sum_{r\in R} p^{(n)}(t = i, b = j|r)\frac{r-\nu_i^{(n)}}{\mu_j^{(n)}}}{\sum_{j=1}^{M} \sum_{r\in R} p^{(n)}(t = i, b = j|r)} \tag{43}$$

$$\kappa_i^{(n+1)} = \left( \frac{\sum_{j=1}^{M} \sum_{r\in R} p^{(n)}(t = i, b = j|r) \left( \frac{r-\nu_i^{(n)}}{\lambda_i^{(n)}} - \mu_j^{(n)} \right)^2}{\sum_{j=1}^{M} \sum_{r\in R} p^{(n)}(t = i, b = j|r)} \right)^{\frac{1}{2}} \tag{44}$$

The advantage of the 6tie-Mix$^2$ model over the 5tie-Mix$^2$ model is that it can model differences in the smoothness between different transcripts. However, the increased number of parameters can also lead to bad model convergence and should therefore only be used if the NGS data exhibits the variance in smoothness implied by the 6tie-Mix$^2$ model.

### 3.2.5 Higher order Mix$^2$ models

In the previous sections the means $\mu_{i,j}$ and standard deviations $\sigma_{i,j}$ of the Gaussians $p_{trans}$ (r|t=i,b=j) were derived from the $\mu_j$ and $\sigma_j$ via the transcript specific affine linear transformations of $\lambda$ given by equations (30) and (31). This section generalizes this concept and allows the $\mu_{i,j}$ and $\sigma_{i,j}$ to be generated by polynomials in $\lambda$, i.e.

$$\mu_{i,j} = \nu_i + \sum_{k=1}^{K} \mu_j(k)\lambda^k \qquad (45)$$

$$\sigma_{i,j} = \sum_{k=0}^{K} \sigma_j(k)\lambda^k \qquad (46)$$

The EM update formula for $\mu_j(k_0)$ is given by the following expression.

$$\mu_j(k_0)^{(n)} = \frac{\sum_{i=1}^{N} \sum_{r \in R} p^{(n)}(t=i, b=j|r)\lambda_i^{k_0} \frac{r - \sum_{k=1, k \neq k_0}^{K} \mu(k)\lambda_i^k - \nu_i}{\sigma_{i,j}^2}}{\sum_{i=1}^{N} \sum_{r \in R} p(t=i, b=j|r)\frac{\lambda_i^{2k_0}}{\sigma_{i,j}^2}} \qquad (47)$$

The EM update formulas for the remaining model parameters lead to non-linear equations which can themselves only be solved by iterative procedures.

Example 4: Experiments on scaled and shifted transcript specific probability distributions

In the experiments in this section, the probability $p_{trans}(r|t = i, b = j)$ is factorized as follows

$$p_{trans}(r|t=i, b=j) = p_{trans}(s(r)|t=i, b=j)p_{trans}(l(r)|t=i, b=j, s(r)) \qquad (48)$$

where $s(r)$ and $l(r)$ are the start and length of fragment r. In addition, the probability $p_{trans}(l(r)|t = i, b = j, s(r))$ is assumed to depend only on $s(r)$ and the length of the transcript $l(t)$. Therefore (48) reduces to

$$p_{trans}(r|t=i, b=j) = p_{trans}(s(r)|t=i, b=j)p_{trans}(l(r)|l(t), s(r)) \qquad (49)$$

As a consequence, the mixture model (16) is a mixture model for the start site distribution $p_{trans}(s(r)|t = i)$ multiplied by the probability distribution for the fragment length, i.e.

$$p_{trans}(r|t=i) = p_{trans}(l(r)|l(t), s(r)) \sum_{j=1}^{M_i} \beta_{i,j} p_{trans}(s(r)|t=i, b=j) \qquad (50)$$

Thus, the convergence of the model to the correct $p_{trans}(r|t = i)$ can be evaluated by checking the convergence of the distributions $\sum_{j=1}^{M_i} \beta_{i,j} p_{trans}(s(r)|t=i, b=j)$ to the correct start site distributions and the convergence of the $\alpha_i$ to the correct abundances.

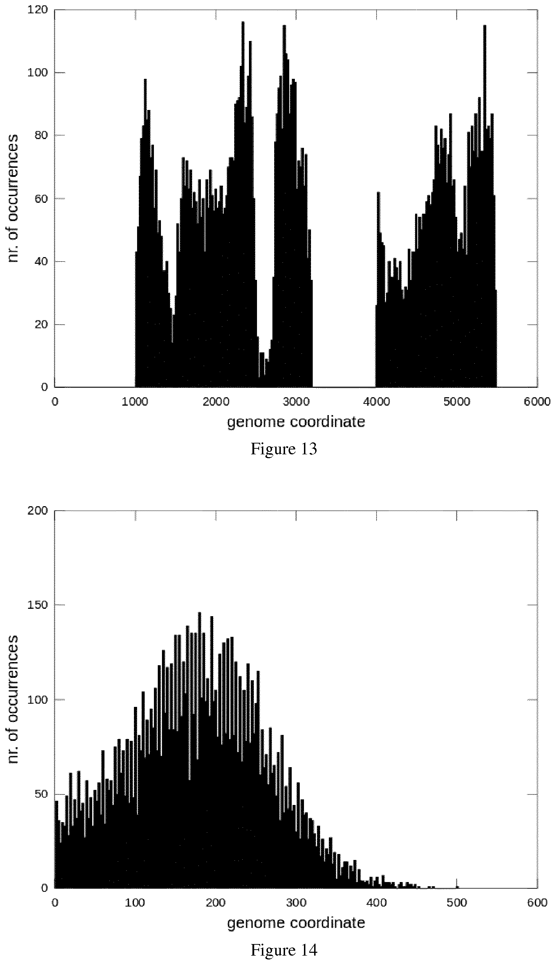

Two types of experiments are discussed in this section. The first type is a detailed analysis of the convergence of the Mix$^2$ model for a set of 10000 fragments where the fragment start sites were randomly drawn from a superposition of the p(r|t=i) in Figure 9 with weights $\alpha_1$=0.28, $\alpha_2$=0.32 and $\alpha_3$=0.4. In addition, for each fragment start site s(r) a fragment length was randomly drawn from a Gaussian with a mean of 200 and a standard deviation of 80 normalized to the possible lengths of fragments starting at s(r), i.e. 1,...,1(t)-s(r)+1. The histograms of the sampled start sites and fragment lengths are shown in Figure 13 and 14. As can be seen, the start site histogram follows roughly the shape of the distribution in Figure 10, whereas the fragment length histogram follows the shape of a Gaussian.

The second type of experiment in this section repeats the first type for 60 different sets of weights $\alpha_1, \alpha_2, \alpha_3$ and compares the $\alpha_1, \alpha_2, \alpha_3$ estimated by the Mix$^2$ model to the true weights. The former experiments provide a detailed insight into the convergence of the Mix$^2$ models and their parameters, while the latter experiments focus on the overall accuracy of the weights $\alpha_1, \alpha_2, \alpha_3$ estimated by the Mix$^2$ model. The set of 60 weights where chosen by setting $\alpha_3$ to each of the values 0.2, 0.4, 0.6, 0.8. For each value of $\alpha_3$, 15 different values of $\alpha_1$ were chosen in equidistant intervals between 0 and 1-$\alpha_3$ and $\alpha_2$ was set to $\alpha_2=1-\alpha_1-\alpha_3$. For each set of weights the Kullback-Leibler (KL) divergence was calculated between the true and the estimated weights and the average of the KL divergence over all sets of weights was used as a measure to quantify the accuracy of Cufflinks and the Mix$^2$ model.

4.1 The 1tie-Mix$^2$ model

4.1.1 Convergence of the 1tie-Mix$^2$ model for $\alpha_1$=0.28, $\alpha_2$=0.32, $\alpha_3$=0.4

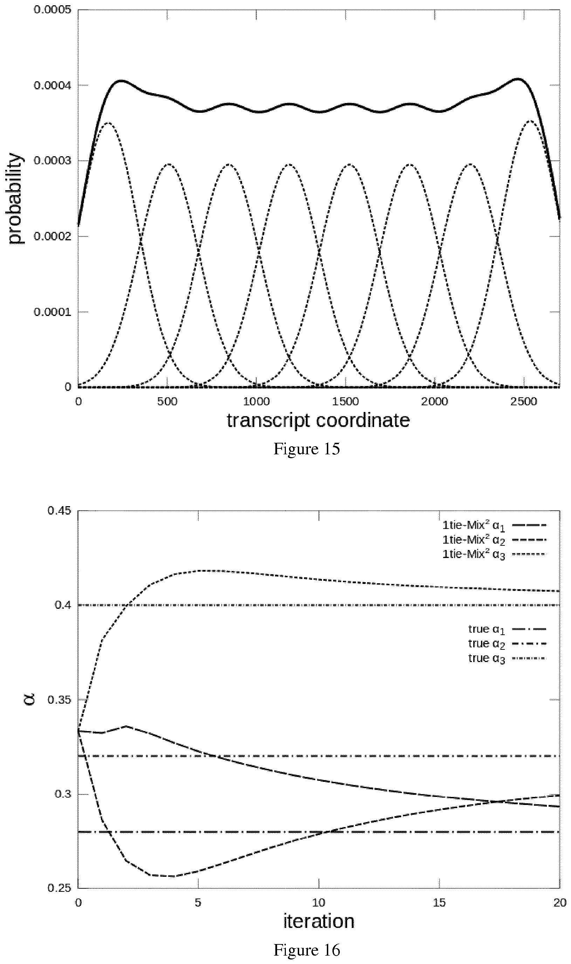

As mentioned before, the data used to estimate the models in this section was generated by sampling the fragment start sites from a superposition of the $p(r|t = i)$ in Figure 9 with weights $\alpha_1 = 0.28$, $\alpha_2 = 0.32$, $\alpha_3 = 0.4$, while the fragment lengths were sampled from a renormalized Gaussian with a mean of 200 and a standard deviation of 80. The histograms of these sampled data set are shown in Figure 13 and Figure 14. The 1tie-Mix$^2$ model in this section uses 8 building blocks $b_{i,j}(s(r))$, which were chosen to be Gaussians. The means of the Gaussians are distributed evenly across the length of the transcript, starting at a position of $\frac{l(t=i)}{2*8}$ and progressing in steps of $\frac{l(t=i)}{8}$, where both numbers were rounded to the nearest integer. Thus for transcript 2 with a length of 2702 bp's, for instance, this results in means at 169, 507, 845, 1183, 1521, 1859, 2197, 2535. The standard deviation of each Gaussian was set to be equal to the first mean. Hence for transcript 2 the standard deviation of each Gaussian was 169. The initial $\beta_j$ in the model were set to $\frac{1}{8}$. The resulting initialization of $p(r|t = i)$ for transcript 2 together with the weighted blocks $\beta_j p(s(r)|t = i, b = j)$ is depicted in Figure 15. The first and last block are a bit higher than the ones within the transcript. This is due to the fact that these Gaussians are missing part of their tail and their normalizing constant is therefore higher than that of the other Gaussians.

The initial abundances in the model were set to $\alpha_1=\alpha_2=\alpha_3=1/3$. The EM algorithm was applied until either the difference between the $\alpha_i$ and $\beta_j$ between subsequent iterations was below 0.001 or the increase in the log likelihood between subsequent iterations was below 0.5. With these termination conditions the EM algorithm converged after 20 iterations.

Table 2: Alphas and Kullback Leibler divergence

| | $\alpha_1$ | $\alpha_2$ | $\alpha_3$ | KL divergence |
|---|---|---|---|---|
| | | | | |
| 1tie-Mix$^2$ | 0.29 | 0.3 | 0.41 | 1.5439e-03 |
| Cufflinks | 0.37 | 0.12 | 0.51 | 1.9647e-01 |

Figure 16 shows the convergence of the $\alpha_i$. The x-axis of this figure represents the iteration of the EM algorithm whereas the y-axis represents the values of the $\alpha_i$. The three curves in Figure 16 all originate from the initial value of 1/3 and converge to values close to the correct solutions, which are indicated by the three horizontal lines in Figure 16. The final values were $\alpha_1$=0.29, $\alpha_2$=0.3, $\alpha_3$=0.41. In comparison, the estimate derived with the Cufflinks model is $\alpha_1$=0.37, $\alpha_2$=0.12, $\alpha_3$=0.51. The numbers are summarized in Table 2 together with the Kullback Leibler (KL) divergence between the true and the estimated values of the $\alpha_i$. The KL divergence is 0 for identical distributions and increases with increasing difference between the distributions. Table 2 shows that the KL divergence between the true and the estimated $\alpha_i$ for the Cufflinks model exceeds the KL divergence of the 1tie-Mix$^2$ model by two orders of magnitude.

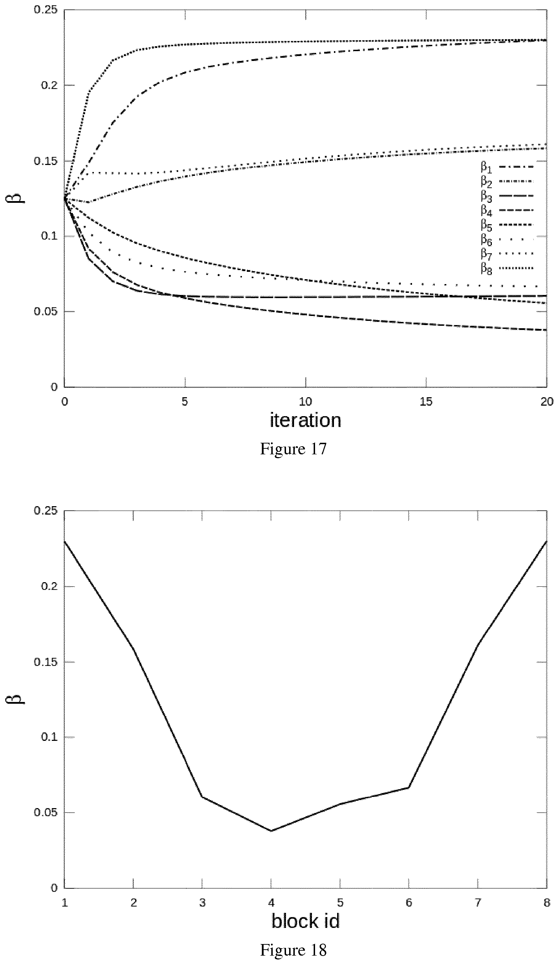

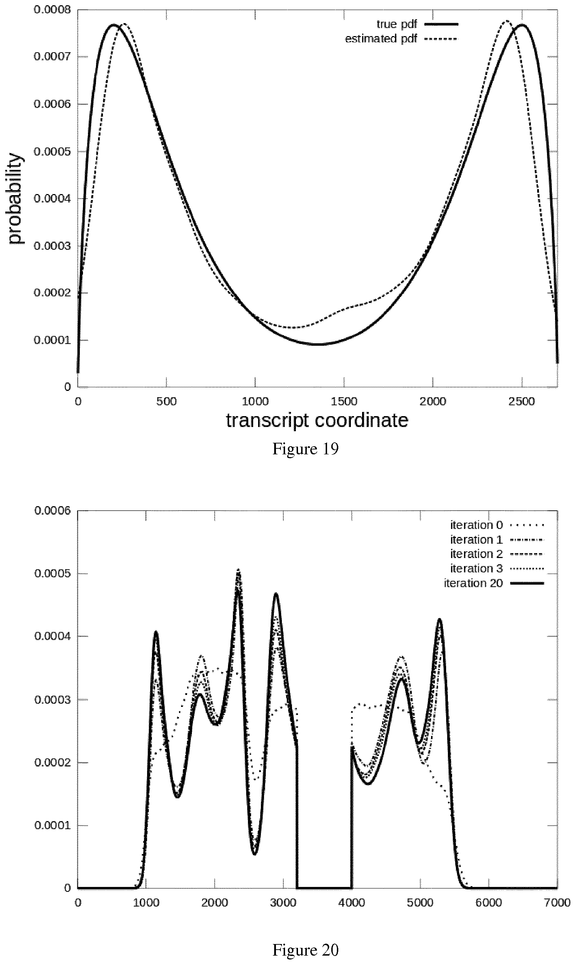

Figure 17 shows the convergence of the 8 betas for the 8 blocks. As before, the x-axis represents the iteration of the EM algorithm and the y-axis represents the values of the $\beta_j$. Since the $\beta_j$ were all initialized to be 1/8, the curves originate from the same position. Since there is no ground truth for the $\beta_j$, as in the case of the $\alpha_i$, their quality can only be evaluated by checking the resulting distributions p(r|t=i). The final $\beta_j$ are shown in Figure 18 where they are plotted against their block id. This shows that the $\beta_j$ roughly follow the shape of the p(r|t=i), which in turn leads to a good approximation of the correct p(r|t=i), as can be seen from Figure 19.

Finally, Figure 20 shows the convergence of the total probability distribution $p_{total}(r)$. This indicates that in terms of overall likelihood the EM algorithm takes the largest step in the direction of the correct solution in the first iteration. Subsequent iterations have a relatively moderate effect on the increase of the overall likelihood. However, in early iterations the $\alpha_i$ are still far from their correct solution. This indicates that also moderate changes in the overall likelihood can lead to meaningful changes of the parameters. As a result, termination conditions of the EM algorithm should be tolerant towards small increases of the total likelihood.

4.1.2 Comparison between the 1tie-Mix$^2$ and Cufflinks models on 60 sets of weights

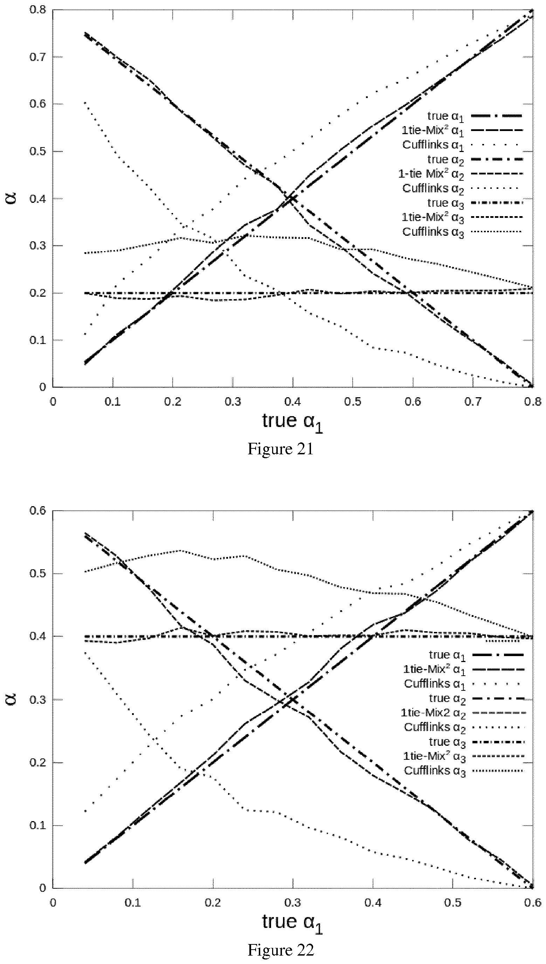

This section discusses experiments with the 60 different sets of weights $\alpha_1,\alpha_2,\alpha_3$, which were chosen according to the procedure described at the beginning of section 4. Thus $\alpha_3$ takes the values 0.2, 0.4, 0.6, 0.8 and $\alpha_1$ and $\alpha_2$ are distributed in equidistant intervals between 0 and 1-$\alpha_3$. For each set of weights p(r|t=i) was initialized for transcript 2 as in Figure 15. The distributions p(r|t=i) for the other two transcripts were initialized accordingly. The EM algorithm was executed until one of the convergence criteria was met. The $\alpha_i$ obtained in the final iteration were chosen as the result of the 1tie-Mix$^2$ model. Similarly the EM algorithm was performed for the Cufflinks model until one of the convergence criteria was met and the $\alpha_i$ from the final iteration were chosen as the result of the Cufflinks model. Figure 21 shows the results of these experiments for $\alpha_3$=0.2. The x-axis of this graph gives the true value of $\alpha_1$ while the y-axis gives the true $\alpha_i$ and the ones estimated by Cufflinks and the 1tie-Mix$^2$ model. The dash-dotted lines in Figure 21 refer to the true $\alpha_i$ whereas the dotted and dashed lines refer to the Cufflinks and 1tie-Mix$^2$ estimates, respectively. Figure 21 shows that for $\alpha_3$=0.2 the 1tie-Mix$^2$ estimate matches the true values of the $\alpha_i$ very well, whereas the Cufflinks estimate is rather poor. Only for $\alpha_2$=0 do the estimates of Cufflinks and the 1tie-Mix$^2$ model coincide. In this situation only transcript one and three are present which are completely separate, as can be seen from Figure 7. In this case therefore the EM algorithm simply counts the number of fragments that are assigned to transcript 1 and 3 and is independent of the shape of the p(r|t=i). Figure 22 shows the estimates of the Cufflinks and 1tie-Mix$^2$ model for $\alpha_3$=0.4. As for $\alpha_3$=0.2 the estimates of the 1tie-Mix$^2$ model are very accurate in comparison to the Cufflinks model. Only for $\alpha_2$=0 do both estimates coincide. A similar picture emerges for $\alpha_3$=0.6 and $\alpha_3$=0.8.

Table 3: Kullback Leibler divergence

| Cufflinks | 1tie-mix$^2$ |
|---|---|
| | |
| 0.12368 | 3.6369e-04 |

As can be seen from Table 3, this leads overall to an average KL divergence of 0.12368 for the Cufflinks model and an average KL divergence of 3.6369e-04 for the 1tie-Mix$^2$ model. Thus, in terms of the average KL divergence the accuracy of the 1tie-Mix$^2$ model exceeds that of the Cufflinks model by 3 orders of magnitude.

4.2 The 5tie-Mix$^2$ model

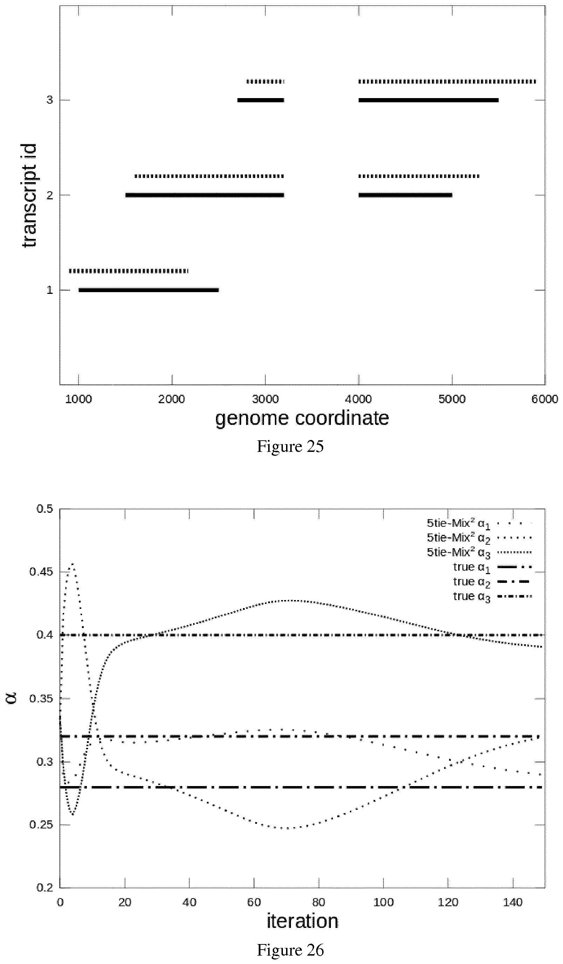

The model in this section estimates transcript specific offset and scaling parameters $\nu_i$ and $\lambda_i$ and does therefore not rely on the correct transcript annotation. Thus, in order to demonstrate the potential of this model it is trained with three incorrect transcript annotations. These annotations can be seen in Figure 25. The solid lines in this figure indicate the correct transcript annotations that are the same as in Figure 7 and Table 1. The dotted lines in Figure 25 show the incorrect transcript annotations that were used to train the models in this section. The exact start and end positions of the exons in the incorrect transcript annotation and their difference from the correct annotation are given in Table 4. The 5tie-Mix$^2$ model in this section uses extended transcript annotations, which start at position 1 and end at position 10000. Thus the extended annotations of transcript 2 and 3 are identical. The $\nu_i$ and $\lambda_i$ of the 5tie-Mix$^2$ model are initialized to conform with the incorrect annotation and are given in Table 5. The $\lambda_i$ of transcript two was chosen to be one and the $\lambda_i$ of transcript one and three are therefore given by 1275/2900=0.4397 and 2300/2900=0.7931. Due to the complicated interaction of the parameters of the 5tie-Mix$^2$ model the likelihood surface of this model has suboptimal local maxima. In order to avoid being trapped in one of these maxima and therefore obtain suboptimal estimates for the $\alpha_i$ it is necessary to initialize the model parameters appropriately. The strategy here was to use the initial values that were obtained for the 1tie-Mix$^2$ model in example 4.1.1 for the incorrect annotation and vary them randomly by a certain amount. In this manner 200 different initial parameter sets were generated on which the EM algorithm was performed until one of the convergence criteria was met, i.e. until either the difference in log-likelihood was below 0.5 or the difference between the $\alpha_i$ and $\beta_j$ was below 0.001 between subsequent iterations. From the 200 obtained results the one with the highest likelihood was chosen as the estimate.

For the other models studied in this section, i.e. the Cufflinks and 1tie-Mix$^2$ models, which cannot correct the incorrect annotation one can expect that the estimated $\alpha_2$ is too high, since transcript two gains more fragments by its increased length than it loses due to its shifted start. Similarly, it can be expected that $\alpha_1$ is more strongly underestimated than $\alpha_3$ since transcript one loses more fragments at its end than transcript three at its start.

Table 4: Incorrect transcript annotation

|  | Exon 1 | Exon 2 | Length |
|---|---|---|---|
|  |  |  |  |
| Transcript 1 | [900, 2174] | NA | 1275 |
| Transcript 2 | [1600, 3200] | [4000, 5298] | 2900 |
| Transcript 3 | [2800, 3200] | [4000, 5898] | 2300 |

Table 5: Correct vs. incorrect transcript annotation. Relative differences and initial $\nu_i$ and $\lambda_i$

|  | Start difference | Length difference | initial $\nu_i$ | initial $\lambda_i$ |
|---|---|---|---|---|
|  |  |  |  |  |
| Transcript 1 | -100 | -226 | 900 | 0.4397 |
| Transcript 2 | +100 | +198 | 1600 | 1 |
| Transcript 3 | +198 | +298 | 2800 | 0.7931 |

4.2.1 Convergence of the 5tie-Mix$^2$ model for $\alpha_1$=0.28, $\alpha_2$=0.32, $\alpha_3$=0.4

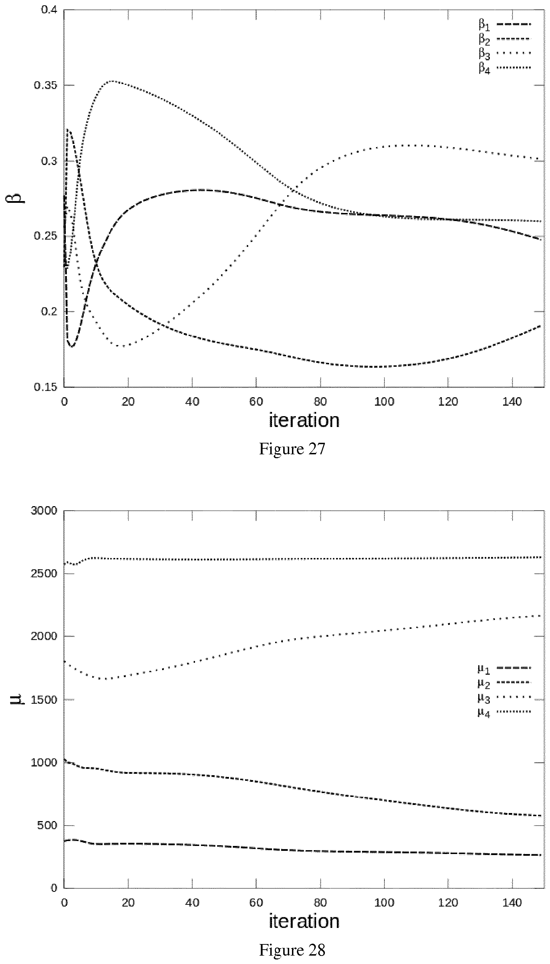

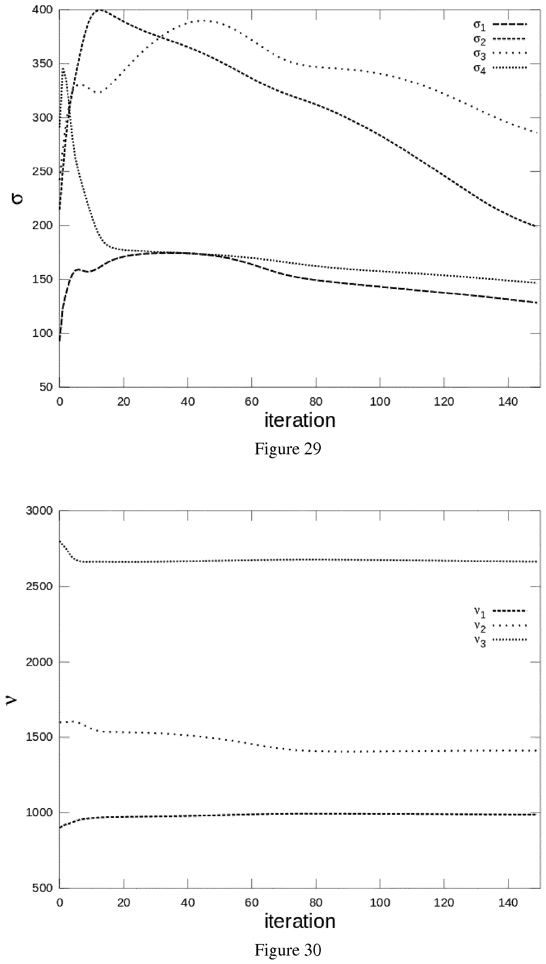

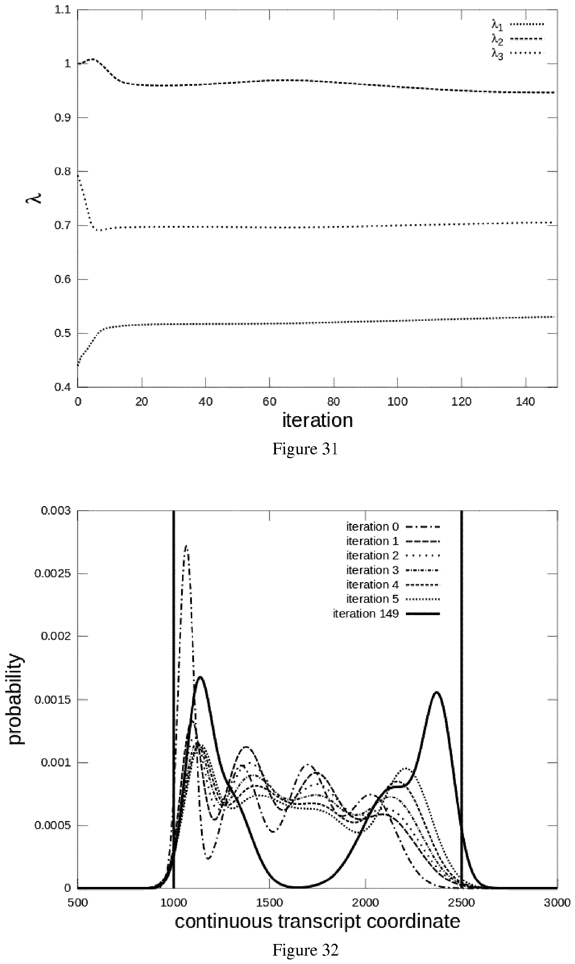

Figure 26 shows the convergence of the $\alpha_i$ for the initial parameter set with the highest likelihood after convergence of the EM algorithm for the weights $\alpha_1$=0.28, $\alpha_2$=0.32, $\alpha_3$=0.4. For this initial parameter set the EM algorithm converged after 149 iterations. The x-axis in Figure 26 gives the iteration of the EM algorithm and the y-axis gives the corresponding $\alpha$. The dotted lines in Figure 26 show the $\alpha_i$ during the course of the EM algorithm, while the horizontal dash-dotted lines show the true $\alpha_i$. Figure 26 indicates that the $\alpha_i$ converge to values very close to the true values. This is also reflected in Table 6, which gives the estimated $\alpha_i$ along with their KL divergence from the correct weights after the final iteration of the EM algorithm. The values of the other model parameters during the EM algorithm are shown in Figures 27, 28, 29, 30 and 31. As previously, the quality of the obtained values of the $\beta_j$, $\mu_j$, $\sigma_j$ can only be evaluated by investigating the resulting p(r|t=i). In comparison, the transcript specific shift and scale parameters $\nu_i$ and $\lambda_i$ give an indication as to whether the final model corrects for the incorrect initial assumption. An increase in $\nu_i$ indicates a shift to the right, while an increase in $\lambda_i$ indicates an increase in length. Figures 30 and 31 therefore indicate that the 5tie-Mix$^2$ model shifts the start of transcript 1 to the right and increases its length, while it shifts the start of transcript 2 and 3 to the left and decreases their length. This is in accordance with the deviation of the incorrect from the correct annotation as shown in Table 5.

Table 6: Alphas and Kullback Leibler divergence

|  | $\alpha_1$ | $\alpha_2$ | $\alpha_3$ | KL divergence |
|---|---|---|---|---|
|  |  |  |  |  |
| 5tie-Mix$^2$ | 0.29 | 0.32 | 0.39 | 4.0270e-04 |
| 1tie-Mix$^2$ | 0.21 | 0.46 | 0.32 | 0.0630711 |
| Cufflinks | 0.2 | 0.5 | 0.31 | 0.0884760 |

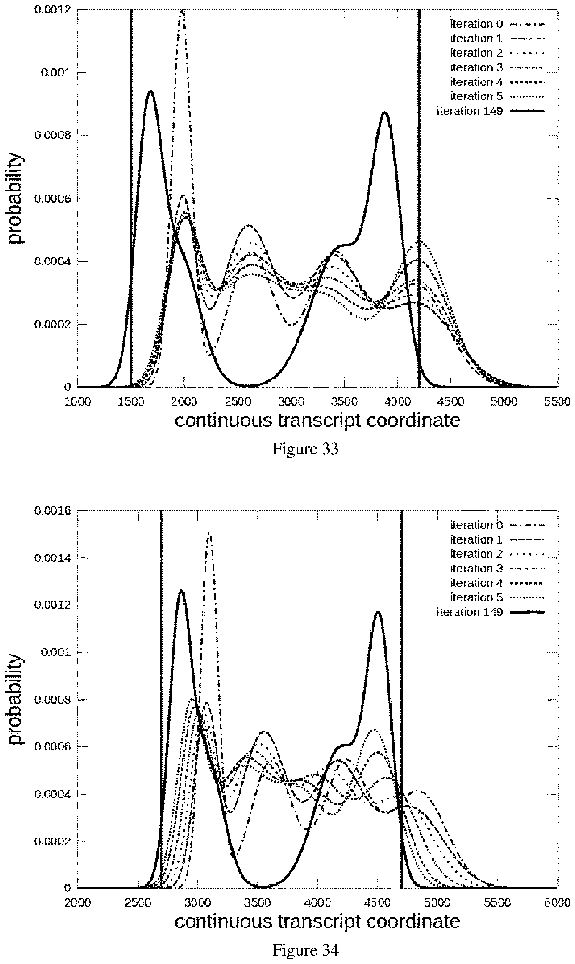

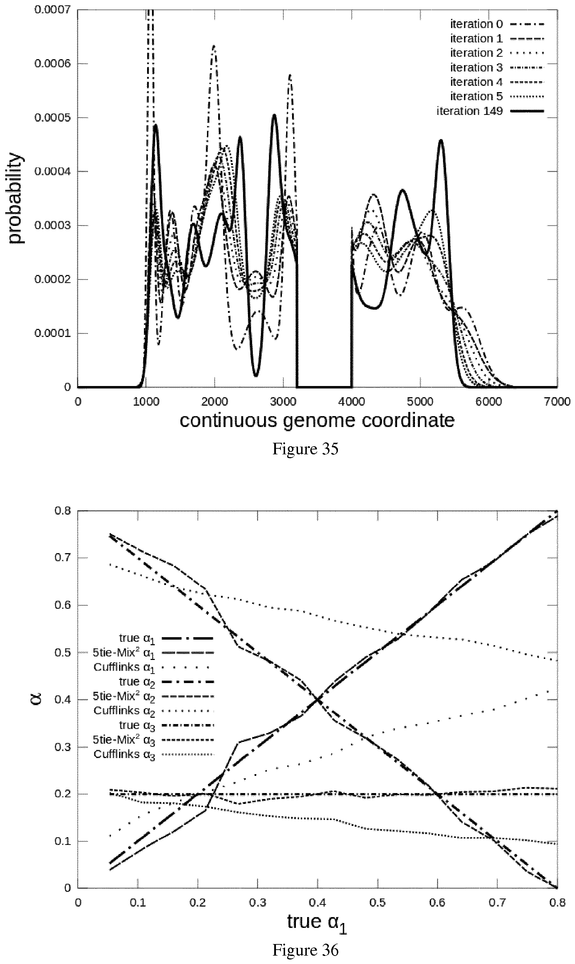

The correction of the incorrect transcript annotation during the EM algorithm can also be seen in Figures 32, 33 and 34 which show the convergence of the $p^{(n)}(r|t = i)$. The vertical lines in these figures indicate the correct start and end in transcript coordinates of the transcript in question. Figure 32 shows the convergence of $p^{(n)}(r|t = i)$ for transcript 1. The dash-dotted line in this figure gives the initial transcript specific pdf $p^{(0)}(r|t = i)$, while the solid line shows $p^{(149)}(r|t = i)$, the result of the EM algorithm after 149 iterations. Figure 32 shows that, in accordance with Figures 30 and 31, $p^{(n)}(r|t = i)$ moves slightly to the right and increases in length during the EM algorithm. The resulting values of transcript start and transcript length in $p^{(149)}(r|t = i)$ are almost perfect. Figure 32 also shows that a major step towards the correct annotation occurs in the first few iterations of the EM algorithm. This is in accordance with Figures 30 and 31, which show that the $\nu_i^{(n)}$ and $\lambda_i^{(n)}$ stay practically constant after 20 EM iterations. Figures 33 and 34 show the convergence of $p^{(n)}(r|t = i)$ for transcript 2 and 3. These figures show that, as for transcript 1, the EM algorithm corrects the initial incorrect transcript annotation by shifting both transcripts to the left and decreasing their length. Finally, Figure 35 shows the convergence of $p_{total}^{(n)}(r)$. As can be seen by the solid line in Figure 35, the final $p_{total}^{(149)}(r)$ is a good approximation to the shape of the histogram in Figure 13.

4.2.2 Comparison between the 5tie-Mix$^2$, 1-tie-Mix$^2$ and Cufflinks models on 60 sets of weights

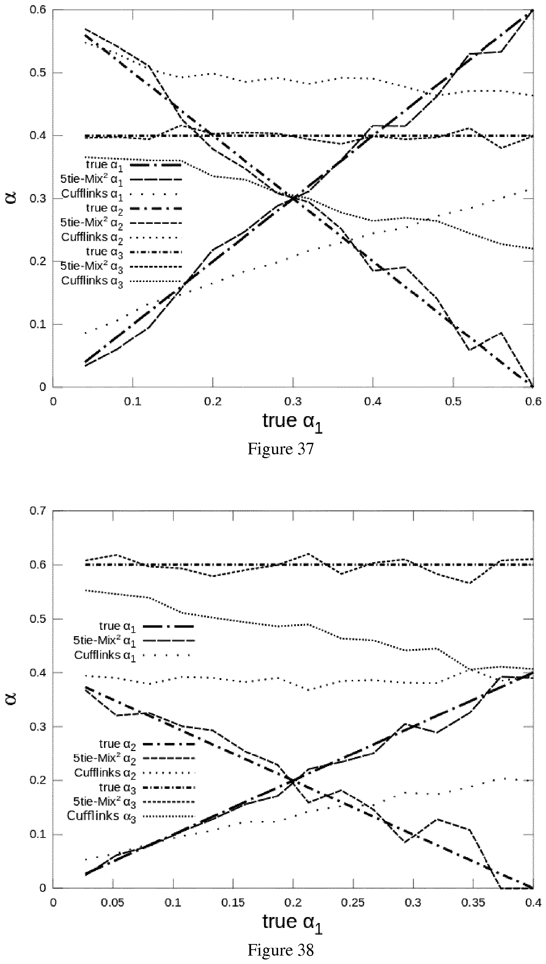

This section discusses experiments with the 60 different sets of weights $\alpha_1$, $\alpha_2$, $\alpha_3$, and the 5tie-Mix$^2$, 1tie-Mix$^2$ and Cufflinks models. The incorrect transcript annotations in Figure 25 were used to initialize the parameters of the 5tie-Mix$^2$ model and as a fixed frame of reference for the 1tie-Mix$^2$ and Cufflinks models. Figure 36 shows the estimated $\alpha_i$ for the 5tie-Mix$^2$ and the Cufflinks models for a true value of $\alpha_3=0.2$. This figure shows a good agreement between the true $\alpha_i$ and the $\alpha_i$ estimated by the 5tie-Mix$^2$ model, whereas the $\alpha_i$ estimated by the Cufflinks model deviate strongly from the correct solution. Only for small values of the true $\alpha_1$ does the Cufflinks model produce estimates which come close to the 5tie-Mix$^2$ model. In this case, transcript one has a very low concentration and therefore only fragments from transcripts two and three play a role in the EM algorithm. These fragments are distributed almost correctly between transcripts two and three by the EM algorithm since the number of fragments that transcript one loses to transcript two due to its incorrect end is negligible. With increasing $\alpha_1$, however, the Cufflinks model seriously overestimates $\alpha_2$ and the underestimation of $\alpha_1$ and $\alpha_3$ become more pronounced. This effect is even stronger in Figure 37 where after a short initial decline the Cufflinks estimate for $\alpha_2$ seems to be almost constant at 0.5. In comparison, the $\alpha_1$ estimated by the 5tie-Mix$^2$ model are again very accurate across the whole range of weights.

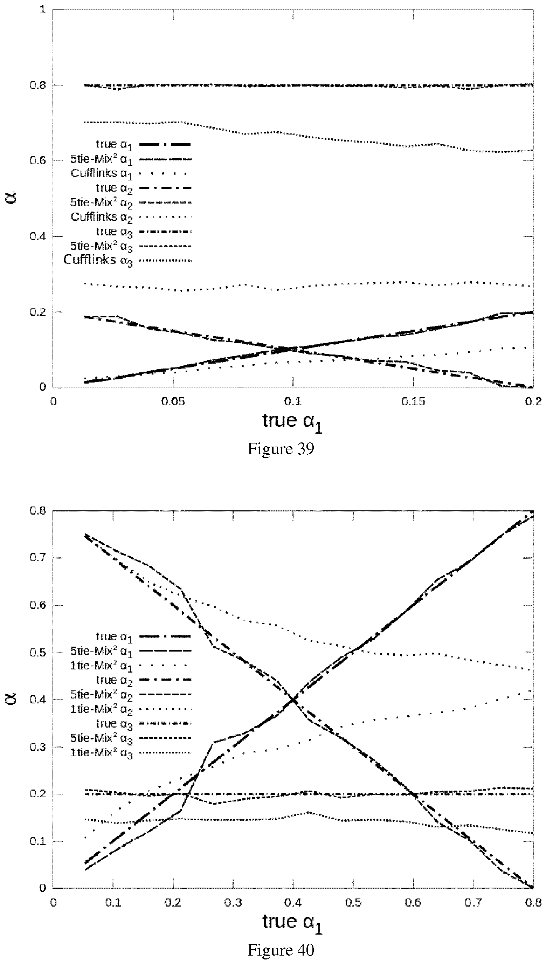

In Figures 38 and 39 the estimates of the 5tie-Mix$^2$ model are again very accurate while the Cufflinks model seriously underestimates $\alpha_1$ and $\alpha_3$. In addition, in Figures 38 and 39 the Cufflinks model yields almost constant estimates for alpha$_2$ of 0.4 and 0.28, respectively. As can be seen from Table 7, over the complete 60 parameter sets the Cufflinks model obtains an average KL divergence of 0.21977 while the 5tie-Mix$^2$ model obtains an average KL divergence of 0.014482. Thus in terms of the average KL divergence the accuracy of the 5tie-Mix$^2$ model exceeds the accuracy of the Cufflinks model by a factor of 15. In fact, the 5tie-Mix$^2$ model is even more accurate than the Cufflinks model with the correct annotation which, according to Table 3, yields an average KL divergence of 0.12368. Thus in terms of the average KL divergence the accuracy of the 5tie-Mix$^2$ model with incorrect transcript annotation exceeds the accuracy of the Cufflinks model with correct annotation by a factor of 8.

Table 7: Average Kullback Leibler divergence for the 5tie-Mix$^2$ and Cufflinks models

| Cufflinks incorrect annotation | 5tie-Mix$^2$ |
|---|---|
|  |  |
| 0.21977 | 0.014482 |

4.2.3 Comparison with the 1par-Mix$^2$ model

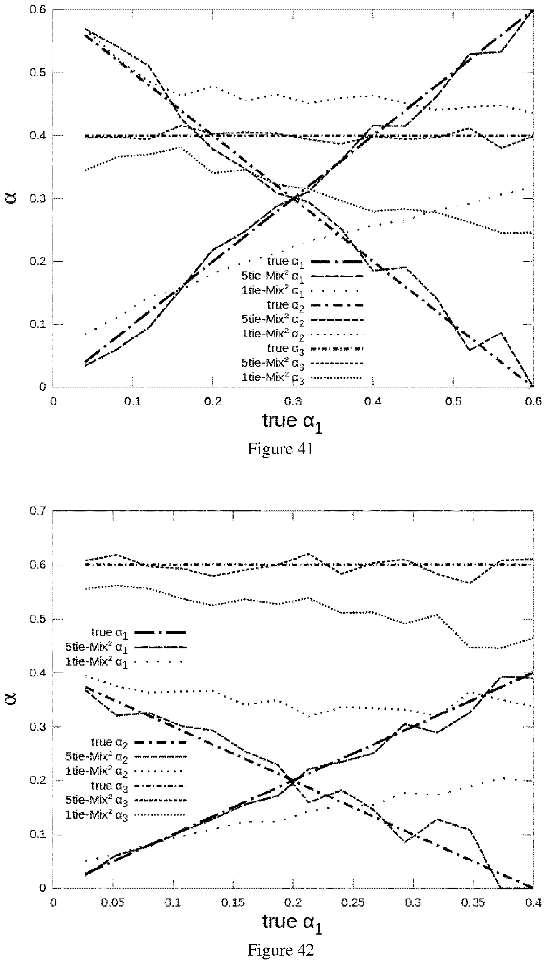

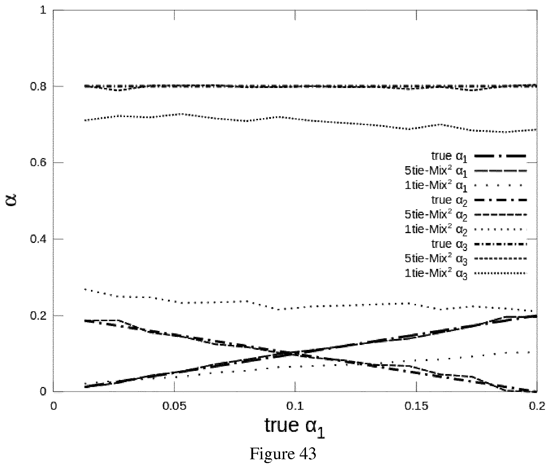

Since the 1tie-Mix$^2$ model was much better than the Cufflinks model in the experiments with correct transcript annotation in Section 4.1.2, it is worth to compare the 5tie-Mix$^2$ model and the 1tie-Mix$^2$ model both with the incorrect transcript annotation. Figure 40 shows the estimated $\alpha_1$ for the 1tie-Mix$^2$ and the 5tie-Mix$^2$ models and a value of the true $\alpha_3$ of 0.2. As in Figure 36, $\alpha_2$ is seriously overestimated by the 1tie-Mix$^2$ model which uses the fixed incorrect transcript annotation. Even though the estimates for the $\alpha_i$ of the 1tie-Mix$^2$ model in Figure 40 are slightly more accurate than the estimates of the Cufflinks model in Figure 36, they are still considerably worse than the 5tie-Mix$^2$ model. This trend is also exhibited in Figures 41, 42 and 43 which show that the estimates for the 1tie-Mix$^2$ model are a small improvement over the estimates for the Cufflinks model but are considerably worse than the estimates of the 5tie-Mix$^2$ model. Overall, this leads to the average KL divergence for the 1tie-Mix$^2$ model in Table 8, which shows that in terms of the KL divergence the accuracy of the 5tie-Mix$^2$ model is about 12 times higher than that of the 1tie-Mix$^2$ model. In summary, the experiments in this section show that in order to obtain reliable estimates for the $\alpha_i$ in the absence of a correct transcript annotation it is important to use a model that is capable of learning the correct transcript locations.

Table 8: Average Kullback Leibler divergence for the 5tie-Mix$^2$ and 1tie-Mix$^2$ models

| 1tie-Mix$^2$ | 5tie-Mix$^2$ |
|---|---|
|  |  |
| 0.18106 | 0.014482 |

Example 5: Conclusions

The experiments in the last section compared the 1tie-Mix$^2$ and the 5tie-Mix$^2$ model with the Cufflinks model both with correct and incorrect transcript annotations. The results of the experiments on the set of 60 parameter sets $\alpha_1$, $\alpha_2$, $\alpha_3$ are summarized in Table 9. These results show that with a correct transcript annotation the 1tie-Mix$^2$ model is far superior to the Cufflinks model, while with an incorrect transcript annotation the 5tie-Mix$^2$ model outperforms both the 1tie-Mix$^2$ and the Cufflinks model. This implies that the use of the appropriate Mix$^2$ model improves the accuracy of the abundance estimates considerably.

Table 9: Average Kullback Leibler divergence for the 1tie-Mix$^2$, the 5tie-Mix$^2$ and Cufflinks models for correct and incorrect annotations

| Cufflinks | | 1tie-Mix$^2$ | | 5tie-Mix$^2$ |
|---|---|---|---|---|
|  |  |  |  |  |
| correct | incorrect | correct | incorrect | extended |
| 0.12368 | 0.21977 | 0.0013911 | 0.18106 | 0.014482 |

Example 6: Comparison with prior art

Cufflinks is a program that implements methods for transcript assembly and transcript abundance estimation. A detailed description of the implementation can be found in Trapnell et al., 2010. In Cufflinks, transcript abundances, which correspond to the $\alpha_1$ according to the invention, are estimated via the factorization in equation (13), where $p_{trans}(s(r)|t=i,l(r))$ is uniform and $p_{trans}(l(r)|t=i)$ is independent of transcript t=i. In contrast to the Mix$^2$ model, Cufflinks does not learn the distribution of the fragment start sites s(r) from the data and depends furthermore on the availability of a correct transcript annotation.

In Roberts et al., 2011, which implements an extension to the Cufflinks abundance estimation, $p_{trans}(r|t=i)$ is factorized as in equation (13) and $p_{trans}(s(r)|t=i,l(r))$ is a normalized product of sequence specific and positional weights, which are defined for each position in transcript coordinates. This results in a large number of parameters that need to be trained even for a single transcript. In order to make the model in the experiments in Roberts et al., 2011 computationally tractable the positional weights have to be restricted to step functions only 5 of which are estimated and shared between all transcripts. In contrast to the model in Roberts et al., 2011, the inventive model does not estimate all the probabilities $p_{trans}(s(r)|t=i)$ individually, but the parameters of the mixtures (16). As a result, far fewer parameters need to be estimated for the inventive model and its parameter estimation is therefore more robust and computationally tractable than that of the model in Roberts et al., 2011. In addition, the model in (Roberts et al., 2011) requires accurate starting estimates for the transcript abundances and its weights are therefore estimated on single isoform genes. Hence the model in Roberts et al., 2011 is not applicable to the data used in the experiments shown herein. The inventive model, on the other hand, does not require accurate starting values for the transcript abundances and can therefore be trained on multi-isoform genes. In addition, the model in Roberts et al., 2011 requires a correct transcript annotation unlike the inventive model.

In Wu et al., 2011 the bias in the distribution of fragments is modeled for the exons of a gene by assigning a single weight to each exon and transcript. In contrast to the inventive model, this restricts the $p_{trans}(s(r)|t=i)$ to be constant on each exon. In addition, the weights in Wu et al., 2011 are estimated partly by heuristic methods outside a probabilistic framework. The work in Wu et al., 2011 further differs from the inventive model in that it uses Poisson distributions for the probability of read counts on exons, whereas the inventive model uses a mixture model for the probability of a fragment given a transcript. In addition, the work in Wu et al., 2011 relies on correct transcript annotations.

The work in Li et al., 2010 corrects for a sequence specific bias and is therefore similar to the sequence specific weights in Roberts et al., 2011. It also resembles the work in Wu et al., 2011 in its usage of Poisson distributions to model count data. In contrast to Wu et al., 2011, the count data in Li et al., 2010 are not exon based but is given by the coverage. The work in Li et al., 2010 differs from the inventive model and the models in Roberts et al., 2011 and Wu et al., 2011 in that it does not estimate the transcript abundances.

In Glaus et al., 2012 a Bayesian model is used for the probability of observing reads generated by a transcript. This model uses the same sequence specific and positional weights as Roberts et al., 2011 and a Gibbs sampling procedure to estimate the transcript abundances. Thus, in comparison to the inventive model, the parameter estimation in Glaus et al., 2012 is performed within a Bayesian rather than a maximum likelihood framework. The model in Glaus et al., 2012 also differs from the inventive model in that it does not use any mixtures for the p(r|t=i) and requires correct transcript annotations.

The models in Li et al., 2010 & 2011 are Bayesian models with a structure similar to the model in Glaus et al., 2012. In contrast to Glaus et al., 2012, however, the transcript abundances in Li et al., 2010 & 2011 are trained within a maximum likelihood framework, in particular with the EM algorithm. The remaining model parameters, such as p(s(r)|t=i), are either derived in advance or are estimated with heuristic methods. This is in contrast to the inventive model which estimates all its parameters within a maximum likelihood framework. In addition, the models in Li et al., 2010 & 2011 do not use mixtures to model the $p_{trans}$(r|t=i) and require correct transcript annotations.

According to the invention the expectation maximization algorithm was used to estimate the inventive model. The general framework of the expectation maximization algorithm was developed in Dempster et al., 1977, while the concrete EM update formulas for the inventive model were derived as described herein.

Example 7: Transcript group tying with the Mix$^2$ model

It was mentioned in the paragraph following equation (20) that tying the parameters of the Mix$^2$ model implies a certain similarity between the $p(r|t=i)$ for different transcripts $t=i$. Therefore, parameters of the Mix$^2$ model should only be tied between transcripts $t=i$, which exhibit this similarity. If transcripts are separated into different groups such that only the transcripts within a group share their parameters, then the EM update formulas of the Mix$^2$ model (27), (36) and (37) need to be modified. In the following, each transcript $t=i$ has an associated group $g = k$ which can be retrieved via the function $G(i)=k$. Then the EM update formulas of the parameters $\beta_{k,j}, \mu_{k,j}$ and $\sigma_{k,j}$ within group $g=k$ are given as follows.

Using the Lagrange method to enforce the constraint

$$\sum_{j=1}^{M} \beta_{k,j} = 1 \qquad (51)$$

and taking the derivative with respect to $\beta_{k,j}$ leads to

$$\sum_{i:G(i)=k} \sum_{r \in R} p(t=i, b=j \mid r) + \beta_{k,j}\lambda = 0 \qquad (52)$$

which after some rearrangement results in

$$\beta_{k,j}^{(n+1)} = \frac{\sum_{r} p^{(n)}\big(g=k, b=j \mid r\big)}{\sum_{r} p^{(n)}\big(g=k \mid r\big)} \qquad (53)$$

where

$$p(g=k, b=j \mid r) = \sum_{i:G(i)=k} p(t=i, b=j \mid r) \qquad (54)$$

For the remaining parameters which are tied between transcripts, i.e. $\mu_{k,j}$ and $\sigma_{k,j}$, the update formulas (36) and (37) need to be modified by replacing the sum over the complete set of transcripts $t=1,...,N$ with the sum over the transcripts within the group $g = k$, i.e.

$$\mu_{k,j}^{(n+1)} = \frac{\displaystyle\sum_{i:G(i)=k}\sum_{r\in R} p^{(n)}(t=i,b=j\,|\,r)\frac{r-v_i^{(n)}}{\lambda_i^{(n)}}}{\displaystyle\sum_{r\in R} p^{(n)}(g=k,b=j\,|\,r)} \qquad (55)$$

$$\left(\sigma_j^{(n+1)}\right)^2 = \frac{\displaystyle\sum_{i:G(i)=k}\sum_{r\in R} p^{(n)}(t=i,b=j\,|\,r)\left(\frac{r-v_i^{(n)}}{\lambda_i^{(n)}}-\mu_j^{(n)}\right)^2}{\displaystyle\sum_{r\in R} p^{(n)}(g=k,b=j\,|\,r)} \qquad (56)$$

Placing each transcript in a genomic locus into its own group removes all parameter tying and leads, as mentioned before, to inaccurate estimates for fragment distributions and transcript abundances. When placing transcripts into different groups it is therefore important to strike the right balance between separation of dissimilar fragment distributions $p(r|t=i)$ and retaining the tying of a sufficient number of parameters to ensure the stability of the Mix$^2$ model. A sensible requirement, for instance, is that each group contains at least two transcripts. Alternatively, in the presence of three or more groups it is sensible to require that at most one group contains a single transcript.

Example 8: Training the fragment length distribution with the Mix$^2$ model

In the experiments discussed in section 4 the transcript specific fragment probability $p_{trans}(r|t=i,b=j)$ was factorized as in (48) and the probability distribution of the fragment lengths $p_{trans}(l(r)|t=i,b=j,s(r))$ was assumed to depend only on the fragment start $s(r)$ and the length $l(t=i)$ of transcript $t=i$. In addition, $p_{trans}(l(r)|l(t=i),s(r))$ was assumed to be given. These assumptions are unnecessary if $p_{trans}(l(r)|t=i,b=j,s(r))$ is estimated from the data set $R$, which can be done within the framework of the Mix$^2$ model. For this purpose, as for the distributions of the fragment starts $s(r)$, $p_{trans}(l(r)|t=i,b=j,s(r))$ is written as a mixture, i.e.

$$p_{trans}(l(r)\,|\,t=i,bs=j,s(r)) = \sum_k \gamma_k\, p_{trans}(l(r)\,|\,t=i,bs=j,bl=k,s(r)) \qquad (57)$$

where $bs=j$ is the hidden variable that was previously denoted by $b=j$. Here $bs$ is a mnemonic for "building block of fragment start", while the hidden variable $bl$ is a mnemonic for "building block of fragment length". It is sensible to assume that $p_{trans}(l(r)|t=i,bs=j,s(r))$ is independent of $bs=j$ and thus (57) reduces to

$$p_{trans}(l(r)\,|\,t=i,s(r)) = \sum_k \gamma_k\, p_{trans}(l(r)\,|\,t=i,bl=k,s(r)) \qquad (58)$$

Combining (15), (48) and (58) yields the following expression for the probability of a fragment

$$p_{total}(r) = \sum_{i,j,k} \alpha_i \beta_j \gamma_k\, p_{trans}(r\,|\,t=i,bs=j,bl=k) \qquad (59)$$

where the following holds

$$p_{trans}(r\,|\,t=i) = \sum_{j,k} \beta_j \gamma_k\, p_{trans}(s(r)\,|\,t=i,bs=j)\, p_{trans}(l(r)\,|\,t=i,bl=k,s(r)) \qquad (60)$$

Hence (59) is a Mix$^2$ model where the mixture weights of $p_{trans}(r|t=i)$ are the product of $\beta_j$ and $\gamma_k$ and the mixture components of $p_{trans}(r|t=i)$ are the product of the conditional probability distributions in (60).

In addition to (15) and (25), the following can be derived from (59)

$$p_{total}(r) = \sum_k \gamma_k p_{trans}(r \mid bl = k) \tag{61}$$

And as a consequence, similar to (22) and (27), $\gamma_k$ can be estimated with the EM algorithm as follows

$$\gamma_k^{(n+1)} = \frac{1}{|R|} \sum_{r \in R} p_{trans}^{(n)}(bl = k \mid r) \tag{62}$$

where

$$p_{trans}(bl = k \mid r) = \frac{p_{trans}(r, bl = k)}{p_{trans}(r)} \tag{63}$$

and

$$p_{trans}(r, bl = k) = \sum_{i,j} \alpha_i \beta_j \gamma_k p_{trans}(r \mid t = i, bs = j, bl = k) \tag{64}$$

For the fragment length building blocks $p_{trans}(l(r)|t=i, bl = k, s(r))$ one can, for instance, use Gaussians whose means are equidistantly distributed either between $s(r)$ and $l(t=i)$ or 1 and $l(t=i)$ and which are either normalized within a discrete or a continuous one-dimensional probability space. If distributions on a continuous one-dimensional probability space are chosen then their internal parameters such as means, standard deviations, shift and scale parameters can be estimated similarly to these parameters for the probability distributions of the fragment starts $p_{trans}(s(r)|t = i)$.

Example 9: Estimating $v_i$ with fixed transcript end point

The 5tie-Mix$^2$ model estimates the shift and scale parameters $v_i$ and $\lambda_i$. If $v_i$ is set to 0 and is not updated during the EM algorithm, then the start of transcript $t=i$ remains unchanged. If the end point of a transcript is to be fixed, the 5tie-Mix$^2$ model needs to be modified slightly.

If $L_i$ is the length of transcript $t=i$ according to the annotation then for a fixed end point of transcript $t=i$ the following holds

$$\lambda_i = \frac{L_i - v_i}{L_i} \tag{65}$$

This yields the following EM update formula for $v_i$

$$\left(v_i^{(n+1)}\right)^2 \sum_{r \in R} p^{(n)}(t = i \mid r)$$
$$- v_i^{(n+1)} \sum_{j=1}^{M} \sum_{r \in R} p^{(n)}(t = i, b = j \mid r) \left( 2L_i + \frac{L_i^3 - L_i^2(r - \mu_j)}{\sigma_j^2} \right)$$
$$+ \sum_{j=1}^{M} \sum_{r \in R} p^{(n)}(t = i, b = j \mid r) \left( L_i^2 + \frac{L_i^3(r - \mu_j) - L_i^2(r - \mu_j)^2}{\sigma_j^2} \right) \tag{66}$$
$$= 0$$

From which $\lambda_i^{(n+1)}$ can be derived according to (65), i.e.

EP 2 943 906 B1

$$\lambda_i^{(n+1)} = \frac{L_i - v_i^{(n+1)}}{L_i} \qquad (67)$$

Example 10: Combining the Mix$^2$ model with other bias models

The Mix$^2$ parameter tying discussed in the experiments in Section 4 implements a model for the positional fragmentation bias, i.e. the bias related to the fragment start within a transcript. Other kinds of bias, like the sequence specific bias, might be described with other models such as the variable length hidden Markov model (VLMM) in [6]. Typically, models for non-positional bias compare the observed frequency of nucleotide sequences to their frequency under the null-hypothesis of unbiased data. This comparison can be used to derive a probability distribution $p(m=c|r)$ over the multiplicity $m=c$ of fragment $r$ in the absence of any bias, given that a single copy of $r$ is observed in the biased data. The distribution $p(m=c|r)$ can then be used to computationally remove the non-positional bias from the data by weighting each fragment $r$ in the EM update formulas of the Mix$^2$ model by the expected multiplicity of $r$. For the EM update formula of the abundances $\alpha_i$, for instance, this leads to

$$\alpha_i^{(n+1)} = \frac{1}{|R|} \sum_{r \in R} \sum_c c \, p(m = c \,|\, r) p^{(n)}(t = i \,|\, r) \qquad (68)$$

It should be noted that the distribution $p(m=c|r)$ cannot be estimated by maximizing the likelihood of the data set $R$ under the Mix$^2$ model, as this would lead to infinite expected values of $p(m=c|r)$. For the combination of the Mix$^2$ model with other bias models, as discussed in this section, it is therefore important to estimate $p(m=c|r)$ outside the maximum likelihood framework of the Mix$^2$ model.

Example 11: Using the Mix$^2$ model to correct FPKM and RPKM values

The concentration of a transcript in RNA-Seq is usually measured with the FPKM (fragments per kilo base pair per million reads) or RPKM (reads per kilo base pair per million reads) measures, where for transcript $t=i$ the latter is given by

$$RPKM\,(t = i) = 10^9 \, \frac{\#reads\ belonging\ to\ transcript\ t = i}{\#total\ reads\ in\ experiment \cdot l(t = i)} \qquad (69)$$

For the FPKM measure the length $l(t=i)$ of transcript $t=i$ in (69) is replaced by an adjusted transcript length $\tilde{l}(t=i)$ [7]. Thus, in order to obtain accurate FPKM and RPKM values it is important to use the accurate transcript length $l(t=i)$ in (69). The 5tie-Mix$^2$ model can correct inaccurate transcript annotations and therefore yields transcript lengths of higher precision, which results in an improved accuracy of the RPKM and FPKM values. Different methods can be employed to obtain an estimate for the transcript length based on the 5tie-Mix$^2$ model. The transcript start can, for instance, be estimated as the position x where

$$\int_{-\infty}^{x} p(s(r)\,|\, t = i) ds(r) = c_s \qquad (70)$$

with $0<c_s<1$. Typically, $c_s$ will be a small positive value such as $c_s$=0.05. Likewise, the transcript end can be estimated as the position where (70) holds with $c_s$ replaced by a value $c_e$ with $0<c_e<1$, which will be close to 1, e.g. $c_e$=0.95. Similarly, (70), with $c_s$ and $c_e$, can be applied to any mixture component $p(r|t=i,b=j)$. In this case, the mixture component used to determine the transcript start will, typically, be concentrated in a region of small $s(r)$, whereas the mixture component used to determine the transcript end will be concentrated in a region of large $s(r)$. Another method to obtain an estimate for the length of transcript $t=i$ from the 5tie-Mix$^2$ model is to multiply the length of the transcript annotation by the $\lambda_i$ after convergence of the Mix$^2$ model.

References

29

[1] A. P. Dempster, N. M. Laird, and D. B. Rubin. Maximum likelihood from incomplete data via the em algorithm. Journal of the Royal Statistical Society, Series B, 39(1):1 38, 1977.

[2] Peter Glaus, Antti Honkela, and Magnus Rattray. Identifying differentially expressed transcripts from RNA-seq data with biological variation. Bioinformatics, 28(13):1721-1728, 2012.

[3] Bo Li and Colin Dewey. RSEM: accurate transcript quantification from rna-seq data with or without a reference genome. BMC Bioinformatics, 12(1):323, 2011.

[4] Bo Li, Victor Ruotti, Ron M Stewart, James A Thomson, and Colin N Dewey. Rna-seq gene expression estimation with read mapping uncertainty. Bioinformatics, 26(4):493-500, Feb 2010.

[5] Jun Li, Hui Jiang, and Wing Wong. Modeling non-uniformity in short-read rates in RNA-seq data. Genome Biology, 11(5):R50+, 2010.

[6] Adam Roberts, Cole Trapnell, Julie Donaghey, John L Rinn, and Lior Pachter. Improving rna-seq expression estimates by correcting for fragment bias. Genome Biol, 12(3):R22, Mar 2011.

[7] Cole Trapnell, Brian A Williams, Geo Pertea, Ali Mortazavi, Gordon Kwan, Marijke J van Baren, Stcvcn L Salzbcrg, Barbara J Wold, and Lior Pachter. Transcript assembly and quantification by RNA-Seq reveals unannotated transcripts and isoform switching during cell differentiation. Nat Biotechnol, 28(5):511 515, May 2010.

[8] Zhengpeng Wu, Xi Wang, and Xuegong Zhang. Using non-uniform read distribution models to improve isoform expression inference in RNA-Seq. Bioinformatics, 27(4):502-508, Feb 2011.

[9] Roberts et al: Genome Biology, 12 (3) (2011): R22

[10] Wen-Ping et al: Genome Biology, 8 (6) (2007): R98

**Claims**

1. Method of estimating transcript abundances comprising the steps of

   a) obtaining transcript fragment sequencing data from a potential mixture of transcripts of a genetic locus of interest,
   b) assigning said fragment sequencing data to genetic coordinates of said locus of interest thereby obtaining a data set of fragment genetic coordinate coverage, said coverage for each genetic coordinate combined forming a coverage envelope curve,
   c) setting a number of transcripts of said mixture, wherein pre-annotated sequence data from the genetic locus of interest is obtained and the number of transcripts is set to at least the number of different isoforms, including splice variants counting as different isoforms, expected from said genetic locus of interest,
   d) pre-setting a probability distribution function of modelled genetic coverage for each transcript i, with i denoting the numerical identifier for a transcript, wherein said probability distribution function is defined by a weight factor $\alpha_i$ of said transcript i multiplied with the sum of at least 2 probability subfunctions j, with j denoting the numerical identifier for a probability subfunction, each probability subfunction j being independently weighted by a weight factor $\beta_{i,j}$, wherein the probability subfunctions j are aperiodic functions, wherein the probability subfunctions j for a transcript are positioned or shifted in the genetic coordinate to cover the entire length of a transcript with a positive value,
   e) adding the probability distribution functions of each transcript to obtain a sum function,
   f) fitting the sum function to the coverage envelope curve thereby optimizing the values for $\alpha_i$ and $\beta_{i,j}$ to increase the fit,
   g) repeating steps e) and f) until a pre-set convergence criterion has been fulfilled, thereby obtaining the estimated transcript abundance for each transcript of the mixture given by the weight factor $\alpha_i$ as optimized after the convergence criterion has been fulfilled;

   wherein steps b)-g) are computer-implemented.

2. The method of claim 1, wherein transcript fragment sequencing data comprises at least 5 transcript fragment sequences.

3. The method of claim 1 or 2, wherein the genetic locus of interest comprises at least two splice variants of one gene or genetic element.

4. The method of any one of claims 1 to 3, wherein the probability subfunction j is constituted of positive values for each genetic coordinate, preferably is a density function.

5. The method of any one of claims 1 to 4, wherein the probability subfunction j is a Gaussian function, a square shape function, or a triangle shape function, especially preferred a Gaussian function.

6. The method of any one of claims 1 to 5, wherein the genetic coordinate corresponds to nucleotide positions in a genome, optionally transformed to omit genetic regions not of interest, wherein preferably said genetic regions not of interest do not contain coverage by said transcript fragment sequencing data.

7. The method of any one of claims 1 to 6, further comprising a step b2) comprising removing genetic coordinate positions with splice junctions from said coverage envelope curve.

8. The method of any one of claims 1 to 7, wherein said fragment genetic coordinate coverage contains the count of at least one nucleotide for each fragment sequence assigned to a genetic coordinate, preferably wherein the at least one nucleotide comprises the fragment start site or the entire fragment sequence.

9. The method of any one of claims 1 to 8, wherein the probability subfunctions for a transcript comprise maxima each at a different genetic coordinate.

10. The method of any one of claims 1 to 9, comprising determining sequence reads of at least one transcript, preferably mRNA, wherein said reads comprise the sequence of fragments of said transcript, to provide said transcript fragment sequencing data.

11. The method of any one of claims 1 to 10, wherein the transcript fragment sequences of said transcript fragment sequencing data have a length of 5 to 800 nucleotides, preferably of 6 to 600 nucleotides, more preferred of 7 to 400 nucleotides, even more preferred of 8 to 200 nucleotides, particularly preferred of 9 to 150 nucleotides, especially preferred of 10 to 100 nucleotides, most preferred of 12 to 70 nucleotides.

12. The method of any one of claims 1 to 11, wherein a full width at half maximum value for each probability subfunction for a transcript i is about identical.

13. A computer readable memory device comprising instructions which, when executed by a computer, cause the computer to carry out a method of any one of claims 1 to 12.

**Patentansprüche**

1. Verfahren zum Schätzen von Transkripthäufigkeiten, die folgenden Schritte aufweisend

   a) Erhalten von Transkriptfragment-Sequenzierungsdaten aus einer potentiellen Mischung von Transkripten eines Genlocus von Interesse,
   b) Zuordnen der Fragment-Sequenzierungsdaten zu genetischen Koordinaten des Locus von Interesse, wodurch ein Datensatz der Fragmentabdeckung der genetischen Koordinaten erhalten wird, wobei die Abdeckung für jede kombinierte genetische Koordinate eine Hüllkurve der Abdeckung bildet,
   c) Festlegen einer Anzahl von Transkripten der Mischung, wobei vorgemerkte Sequenzierungsdaten des Genlocus von Interesse erhalten werden und die Anzahl von Transkripten auf mindestens die Anzahl von unterschiedlichen Isoformen, einschließlich Spleißvarianten, die als unterschiedliche Isoformen zählen, die von dem Genlocus von Interesse erwartet werden, festgelegt wird,
   d) Vorabfestlegen einer Wahrscheinlichkeitsverteilungsfunktion der modellierten genetischen Abdeckung für jedes Transkript i, wobei i die numerische Kennung für ein Transkript bezeichnet, wobei die Wahrscheinlichkeitsverteilungsfunktion durch einen Gewichtungsfaktor $\alpha_i$ des Transkripts i definiert ist, der mit der Summe von mindestens 2 Wahrscheinlichkeitsunterfunktionen j multipliziert wird, wobei j die numerische Kennung für eine Wahrscheinlichkeitsunterfunktion bezeichnet, wobei jede Wahrscheinlichkeitsunterfunktion j unabhängig durch einen Gewichtungsfaktor $\beta_{i,j}$ gewichtet wird, wobei die Wahrscheinlichkeitsunterfunktionen j aperiodische Funktionen sind, wobei die Wahrscheinlichkeitsunterfunktionen j für ein Transkript in der genetischen Koordinate positioniert oder verschoben werden, um die gesamte Länge eines Transkripts mit einem positiven Wert abzudecken,

e) Hinzufügen der Wahrscheinlichkeitsverteilungsfunktionen jedes Transkripts, um eine Summenfunktion zu erhalten,

f) Anpassen der Summenfunktion an die Hüllkurve der Abdeckung, wobei die Werte für $\alpha_i$ und $\beta_{ij}$ optimiert werden, um die Anpassung zu erhöhen,

g) Wiederholen der Schritte e) und f) bis ein vorab festgelegtes Konvergenzkriterium erfüllt wird, wodurch die geschätzte Transkripthäufigkeit für jedes durch den optimierten Gewichtungsfaktor $\alpha_i$ angegebene Transkript der Mischung erhalten wird, nachdem das Konvergenzkriterium verfüllt wurde;

wobei die Schritte b)-g) computerimplementiert sind.

2. Verfahren nach Anspruch 1, wobei die Transkriptfragment-Sequenzierungsdaten mindestens 5 Transkriptfragment-Sequenzen aufweisen.

3. Verfahren nach Anspruch 1 oder 2, wobei der Genlocus von Interesse mindestens zwei Spleißvarianten eines Gens oder eines genetischen Elements aufweist.

4. Verfahren nach einem der Ansprüche 1 bis 3, wobei die Wahrscheinlichkeitsunterfunktion j, die aus positiven Werten für jede genetische Koordinate besteht, vorzugsweise eine Dichtefunktion ist.

5. Verfahren nach einem der Ansprüche 1 bis 4, wobei die Wahrscheinlichkeitsunterfunktion j eine Gaußfunktion, eine rechteckförmige Funktion oder eine dreieckförmige Funktion ist, besonders bevorzugt eine Gaußfunktion.

6. Verfahren nach einem der Ansprüche 1 bis 5, wobei die genetische Koordinate Positionen von Nukleotiden in einem Genom entspricht, welche optional verändert werden, um Genregionen, die nicht von Interesse sind, zu vermeiden, wobei die Genregionen, die nicht von Interesse sind, vorzugsweise keine Abdeckung durch die Transkriptfragment-Sequenzierungsdaten enthalten.

7. Verfahren nach einem der Ansprüche 1 bis 6, zusätzlich einen Schritt b2) aufweisend, welcher ein Entfernen von genetischen Koordinatenpositionen mit Spleißstellen von der Hüllkurve der Abdeckung aufweist.

8. Verfahren nach einem der Ansprüche 1 bis 7, wobei die Fragmentabdeckung der genetischen Koordinaten die Zählung von mindestens einem Nukleotid für jede Fragment-Sequenz, die einer genetischen Koordinate zugeordnet ist, enthält, wobei das mindestens eine Nukleotid vorzugsweise die Fragment-Startstelle oder die gesamte Fragment-Sequenz aufweist.

9. Verfahren nach einem der Ansprüche 1 bis 8, wobei die Wahrscheinlichkeitsunterfunktionen für ein Transkript jeweils an einer unterschiedlichen genetischen Koordinate Maxima aufweisen.

10. Verfahren nach einem der Ansprüche 1 bis 9, welches das Bestimmen der Sequenz-Reads mindestens eines Transkripts, vorzugsweise mRNA, aufweist, wobei die Reads die Sequenz der Fragmente des Transkripts aufweisen, um die Transkriptfragment-Sequenzierungsdaten bereitzustellen.

11. Verfahren nach einem der Ansprüche 1 bis 10, wobei die Transkriptfragment-Sequenzen der Transkriptfragment-Sequenzierungsdaten eine Länge von 5 bis 800 Nukleotiden, vorzugsweise von 6 bis 600 Nukleotiden, mehr bevorzugt von 7 bis 400 Nukleotiden, noch mehr bevorzugt von 8 bis 200 Nukleotiden, insbesondere bevorzugt von 9 bis 150 Nukleotiden, besonders bevorzugt von 10 bis 100 Nukleotiden, am meisten bevorzugt von 12 bis 70 Nukleotiden haben.

12. Verfahren nach einem der Ansprüche 1 bis 11, wobei ein Halbwertsbreitenwert für jede Wahrscheinlichkeitsunterfunktion für ein Transkript i ungefähr identisch ist.

13. Computerlesbare Speichervorrichtung, welche Anweisungen aufweist, die, wenn sie von einem Computer ausgeführt werden, den Computer veranlassen, ein Verfahren nach einem der Ansprüche 1 bis 12 durchzuführen.

**Revendications**

1. Procédé d'estimation d'abondances de transcrits comprenant les étapes de

   a) obtenir des données de séquençage de fragment de transcrit à partir d'un mélange potentiel de transcrits d'un locus génétique d'intérêt,
   b) assigner desdites données de séquençage de fragments à des coordonnées génétiques dudit locus d'intérêt, obtenant de cette façon un ensemble de données de couverture des coordonnées génétiques de fragment, ladite couverture pour chaque coordonnée génétique combinée formant une courbe d'enveloppe de couverture,
   c) régler un nombre de transcrits dudit mélange, dans lequel des données de séquence pré-annotées du locus génétique d'intérêt sont obtenues et le nombre de transcrits est réglé à au moins le nombre des différentes isoformes, y compris des variants d'épissage comptant comme des isoformes différentes, attendues dudit locus génétiques d'intérêt,
   d) Iprérégler une fonction de distribution de probabilités de la couverture génétique modélisée pour chaque transcrit i, i indiquant l'identifiant numérique pour un transcrit, dans lequel ladite fonction de distribution de probabilités est définie par un facteur de pondération $\alpha_i$ dudit transcrit i multiplié par la somme d'au moins 2 sous-fonctions de probabilité j, avec j indiquant l'identifiant numérique pour une sous-fonction de probabilité, chaque sous-fonction de probabilité j étant pondérée indépendamment par un facteur de pondération $\beta_{i,j}$, dans lequel les sous-fonctions de probabilité j sont des fonctions apériodiques, dans lequel les sous-fonctions de probabilité j pour un transcrit sont positionnées ou décalées dans la coordonnée génétique pour couvrir la longueur entière d'un transcrit avec une valeur positive,
   e) ajouter les fonctions de distribution de probabilités de chaque transcrit pour obtenir une fonction de somme,
   f) adapter la fonction de somme à la courbe enveloppe de couverture, optimisant de cette façon les valeurs pour $\alpha_i$ et $\beta_{i,j}$ pour augmenter l'adaptation,
   g) répéter les étapes e) et f) jusqu'à ce qu'un critère de convergence préréglé ait été satisfait, obtenant de cette façon l'abondance de transcrit estimée pour chaque transcrit du mélange donnée par le facteur de pondération $\alpha_i$ tel qu'optimisé après que le critère de convergence a été satisfait ;

   dans lequel les étapes b) à g) sont mises en oeuvre par ordinateur.

2. Procédé selon la revendication 1, dans lequel des données de séquençage de fragment de transcrit comprennent au moins 5 séquences de fragment de transcrit.

3. Procédé selon la revendication 1 ou 2, dans lequel le locus génétique d'intérêt comprend au moins deux variants d'épissage d'un gène ou d'un élément génétique.

4. Procédé selon l'une quelconque des revendications 1 à 3, dans lequel la sous-fonction de probabilité j est constituée de valeurs positives pour chaque coordonnée génétique, de préférence est une fonction de densité.

5. Procédé selon fune quelconque des revendications 1 à 4, dans lequel la sous-fonction de probabilité j est une fonction gaussienne, une fonction en forme de carré, ou une fonction en forme de triangle, est spécialement préférée une fonction gaussienne.

6. Procédé selon l'une quelconque des revendications 1 à 5, dans lequel la coordonnée génétique correspond aux positions nucléotidiques dans un génome, optionnellement transformées pour omettre des régions génétiques non d'intérêt, dans lequel de préférence lesdites régions génétiques non d'intérêt ne contiennent pas de couverture par lesdites données de séquençage de fragment de transcrit.

7. Procédé selon l'une quelconque des revendications 1 à 6, comprenant en outre une étape b2) comprenant retirer des positions de coordonnées génétiques avec des jonctions d'épissage de ladite courbe enveloppe de couverture.

8. Procédé selon l'une quelconque des revendications 1 à 7, dans lequel ladite couverture de coordonnées génétiques de fragment contient le compte d'au moins un nucléotide pour chaque séquence de fragment assigné à une coordonnée génétique, de préférence dans lequel l'au moins un nucléotide comprend le site de départ de fragment ou la séquence entière de fragment.

9. Procédé selon l'une quelconque des revendications 1 à 8, dans lequel les sous-fonctions de probabilité pour un transcrit comprennent des maximums chacun à une coordonnée génétique différente.

10. Procédé selon l'une quelconque des revendications 1 à 9, comprenant déterminer des lectures de séquence d'au moins un transcrit, de préférence d'ARNm, dans lequel lesdites lectures comprennent la séquence de fragments dudit transcrit, pour fournir des données de séquençage de fragment de transcrit.

11. Procédé selon l'une quelconque des revendications 1 à 10, dans lequel les séquences de fragment de transcrit desdites données de séquençage de fragment de transcrit ont une longueur de 5 à 800 nucléotides, de préférence de 6 à 600 nucléotides, de façon davantage préférée de 7 à 400 nucléotides, de façon même davantage préférée de 8 à 200 nucléotides, de façon particulièrement préférée de 9 à 150 nucléotides, de façon spécialement préférée de 10 à 100 nucléotides, de façon préférée entre toutes de 12 à 70 nucléotides.

12. Procédé selon l'une quelconque des revendications 1 à 11, dans lequel une pleine largeur à moitié de la valeur maximale pour chaque sous-fonction de probabilité pour un transcrit i est à peu près identique.

13. Dispositif de mémoire lisible par ordinateur comprenant des instructions qui, lorsqu'elles sont exécutées par un ordinateur, entraine la mise en oeuvre par l'ordinateur d'un procédé selon l'une quelconque des revendications 1 à 12.

Figure 1

Figure 2

Figure 3

Figure 4

Figure 5

Figure 6

Figure 7

Figure 8

Figure 9

Figure 10

Figure 11

Figure 12

Figure 13

Figure 14

Figure 15

Figure 16

Figure 17

Figure 18

Figure 19

Figure 20

Figure 21

Figure 22

Figure 23

Figure 24

Figure 25

Figure 26

Figure 27

Figure 28

Figure 29

Figure 30

Figure 31

Figure 32

Figure 33

Figure 34

Figure 35

Figure 36

Figure 37

Figure 38

Figure 39

Figure 40

Figure 41

Figure 42

Figure 43

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2013110410 A1
- WO 2009085412 A1
- WO 2009091798 A1

### Non-patent literature cited in the description

- **TRAPNELL et al.** *Cufflinks,* 2010
- **A. P. DEMPSTER ; N. M. LAIRD ; D. B. RUBIN.** Maximum likelihood from incomplete data via the em algorithm. *Journal of the Royal Statistical Society,* 1977, vol. 39 (1), 1-38
- **PETER GLAUS ; ANTTI HONKELA ; MAGNUS RATTRAY.** Identifying differentially expressed transcripts from RNA-seq data with biological variation. *Bioinformatics,* 2012, vol. 28 (13), 1721-1728
- **BO LI ; COLIN DEWEY.** RSEM: accurate transcript quantification from rna-seq data with or without a reference genome. *BMC Bioinformatics,* 2011, vol. 12 (1), 323
- **BO LI ; VICTOR RUOTTI ; RON M STEWART ; JAMES A THOMSON ; COLIN N DEWEY.** Rna-seq gene expression estimation with read mapping uncertainty. *Bioinformatics,* February 2010, vol. 26 (4), 493-500
- **JUN LI ; HUI JIANG ; WING WONG.** Modeling non-uniformity in short-read rates in RNA-seq data. *Genome Biology,* 2010, vol. 11 (5
- **ADAM ROBERTS ; COLE TRAPNELL ; JULIE DONAGHEY ; JOHN L RINN ; LIOR PACHTER.** Improving rna-seq expression estimates by correcting for fragment bias. *Genome Biol,* March 2011, vol. 12 (3), R22
- **COLE TRAPNELL ; BRIAN A WILLIAMS ; GEO PERTEA ; ALI MORTAZAVI ; GORDON KWAN ; MARIJKE J VAN BAREN ; STCVCN L SALZBCRG ; BARBARA J WOLD ; LIOR PACHTER.** Transcript assembly and quantification by RNA-Seq reveals unannotated transcripts and isoform switching during cell differentiation. *Nat Biotechnol,* May 2010, vol. 28 (5), 511-515
- **ZHENGPENG WU ; XI WANG ; XUEGONG ZHANG.** Using non-uniform read distribution models to improve isoform expression inference in RNA-Seq. *Bioinformatics,* February 2011, vol. 27 (4), 502-508
- **ROBERTS et al.** *Genome Biology,* 2011, vol. 12 (3), R22
- **WEN-PING et al.** *Genome Biology,* 2007, vol. 8 (6), R98